# EUROPÄISCHE PATENTSCHRIFT

(11) **EP 2 462 224 B1**
(45) Veröffentlichungstag und Bekanntmachung des Hinweises auf die Patenterteilung: **10.05.2017**
(21) Anmeldenummer: 10744875.5
(22) Anmeldetag: 02.08.2010
(51) Int. Cl.: C12N 9/22, C12N 1/21, C12P 21/02

(54) **VERFAHREN ZUR HERSTELLUNG VON NUKLEASEN EINES GRAM-NEGATIVEN BAKTERIUMS UNTER NUTZUNG EINES GRAM-POSITIVEN EXPRESSIONSWIRTES**
METHOD FOR PRODUCING NUCLEASES OF A GRAM NEGATIVE BACTERIA USING A GRAM POSITIVE EXPRESSION HOST
PROCÉDÉ DE FABRICATION DE NUCLÉASES D'UNE BACTÉRIE GRAM NÉGATIF EN UTILISANT UNE CELLULE HOTE D'EXPRESSION GRAM POSITIF

(30) Priorität: 03.08.2009 EP 09009992
(43) Veröffentlichungstag der Anmeldung: 13.06.2012
(73) Patentinhaber: c-LEcta GmbH, 04103 Leipzig (DE)
(72) Erfinder: GREINER-STÖFFELE, Thomas, 04279 Leipzig (DE); SCHÖNERT, Stefan, 04277 Leipzig (DE)
(74) Vertreter: Bülle, Jan
(86) Internationale Anmeldenummer: PCT/EP2010/004709
(87) Internationale Veröffentlichungsnummer: WO 2011/015327

(56) Entgegenhaltungen:
- BIEDERMANN K ET AL: "Fermentation studies of the secretion of Serratia marcescens nuclease by Escherichia coli." APPLIED AND ENVIRONMENTAL MICROBIOLOGY JUN 1990, Bd. 56, Nr. 6, Juni 1990 (1990-06), Seiten 1833-1838, XP002561838 ISSN: 0099-2240 in der Anmeldung erwähnt
- MIYOSHI ANDERSON ET AL: "A xylose-inducible expression system for Lactococcus lactis." FEMS MICROBIOLOGY LETTERS 15 OCT 2004 LNKD- PUBMED:15476967, Bd. 239, Nr. 2, 15. Oktober 2004 (2004-10-15), Seiten 205-212, XP004597825 ISSN: 0378-1097
- KOVACEVIC S ET AL: "Secretion of staphylococcal nuclease by Bacillus subtilis." JOURNAL OF BACTERIOLOGY MAY 1985 LNKD- PUBMED:3921523, Bd. 162, Nr. 2, Mai 1985 (1985-05), Seiten 521-528, XP002601752 ISSN: 0021-9193
- DIEYE Y ET AL: "Design of a protein-targeting system for lactic acid bacteria." JOURNAL OF BACTERIOLOGY JUL 2001, Bd. 183, Nr. 14, Juli 2001 (2001-07), Seiten 4157-4166, XP002561839 ISSN: 0021-9193 in der Anmeldung erwähnt
- KAY TERPE: "Overview of bacterial expression systems for heterologous protein production: from molecular and biochemical fundamentals to commercial systems" APPLIED MICROBIOLOGY AND BIOTECHNOLOGY, SPRINGER, BERLIN, DE, Bd. 72, Nr. 2, 22. Juni 2006 (2006-06-22), Seiten 211-222, XP019422070 ISSN: 1432-0614
- AKIRA NAKAMURA ET AL: 'Gene cloning and characterization of a novel extracellular ribonuclease of Bacillus subtilis' EUROPEAN JOURNAL OF BIOCHEMISTRY Bd. 209, Nr. 1, 01 Oktober 1992, Seiten 121 - 127, XP055169262 DOI: 10.1111/j.1432-1033.1992.tb17268.x ISSN: 0014-2956

## Beschreibung

Die Erfindung betrifft ein Verfahren zur Herstellung einer Nuklease eines gram-negativen Bakteriums oder einer Nuklease-Präparation enthaltend eine Nuklease eines gram-negativen Bakteriums umfassend die Expression der Nuklease in einem gram-positiven Bakterium und die anschließende Sekretion der Nuklease.

Nukleasen sind hydrolytische Enzyme, welche Nukleinsäuren spalten, und haben vielfältige wirtschaftliche Bedeutung. Dabei werden spezifische Nukleasen, wie Restriktionsenzyme, von unspezifischen Nukleasen, wie RNase A, unterschieden. Restriktionsenzyme sind unverzichtbarere Werkzeuge der Molekularbiologie geworden und dienen dazu, verschiedene DNA-Moleküle spezifisch zu spalten, welche mittels Ligasen zu neuen Konstrukten zusammengefügt werden. Unspezifische Nukleasen werden hauptsächlich für den Abbau von Nukleinsäuren in verschiedenen Prozessen genutzt. Spaltet die Nuklease nur DNA, spricht man von einer "DNase", spaltet die Nuklease nur RNA, spricht man von einer "RNase". Ein typischer Vertreter von DNasen ist die DNase I aus dem Pankreas von Säugetieren. Typische Vertreter von RNasen sind zum Beispiel RNase T1 und T2 aus *Aspergillus oryzae* oder RNase A ebenfalls aus Pankreas von Säugetieren.

Neben der Anwendung zur Befreiung von DNA-Proben von RNA durch die Behandlung mit RNasen bzw. der Befreiung von RNA-Proben von DNA durch die Behandlung mit DNasen sind ebenfalls Anwendungen von großem wirtschaftlichem Interesse, bei denen sowohl DNA als auch RNA aus der Probe entfernt werden. Dies betrifft zum Beispiel die Produktion verschiedenster Moleküle durch zellbasierte oder zellfreie, biologische Systeme, bei denen das Produkt nicht aus Nukleinsäuren besteht, wie zum Beispiel im Falle von Proteinen, wie Antikörper oder Enzyme, Polysachariden, Lipiden oder niedermolekularen Stoffen, wie Antibiotika, Stoffwechselend- und Stoffwechselzwischenprodukten oder Chemikalien. Besonders zum Tragen kommt die Notwendigkeit der Entfernung der Nukleinsäuren, wenn die Produktion der Moleküle intrazellulär erfolgt oder bei der Produktion ein Anteil der Produktionszellen lysiert wird. Dadurch werden bei der Präparation der Moleküle auch große Anteile von Nukleinsäuren mit freigesetzt oder sind in der Präparation enthalten, die das gewünschte Molekül verunreinigen oder dessen weitere Reinigung erschweren. Eine ähnliche Problemstellung ergibt sich zum Beispiel bei der Herstellung von Proteinen mit Hilfe von zellfreier, in-vitro Translation. Die Reinigung wird unter anderem dadurch erschwert, dass die Nukleinsäuren die Viskosität der Präparationen in dem Maße erhöhen, dass nachfolgende Schritte wie Filtrationen oder Chromatographien nicht möglich sind.

Es besteht somit ein großes Interesse bei solchen Prozessen, die verunreinigenden Nukleinsäuren zu entfernen oder so weit zu verdauen, dass keine Beeinträchtigung der weiteren Prozessschritte mehr auftritt. Eine Möglichkeit zur Entfernung der Nukleinsäuren besteht in der spezifischen Fällung der Nukleinsäuren mittels verschiedener Agenzien. Eine andere Möglichkeit besteht darin, die Nukleinsäuren durch Nukleasen zu so kleinen Stücken abzubauen, dass die Viskosität der Proben verringert wird und die entstandenen Abbauprodukte durch einfache Methoden wie z. B. Ultrafiltration abgetrennt werden können.

Besonders vorteilhaft für eine Anwendung zur Entfernung von allen Nukleinsäuren, d.h. RNA und DNA, aus verschiedenen Proben ist der Einsatz von Nukleasen, welche sowohl RNA als auch DNA spalten können. Dabei sollte die eingesetzte Nuklease eine hohe Aktivität und ausreichende Stabilität aufweisen. Eine Nuklease, welche diese Eigenschaften erfüllt ist, die Nuklease aus dem gram-negativen Bakterium *Serratia marcescens* [EC 3.1.30.2; SEQ ID 1; Filimonova MN, Balaban NP, Sharipova FP, Leshchinskaia IB, Biokhimiia. 1980, 45(11):2096-104; Filimonova MN, Baratova LA, Vospel'nikova ND, Zheltova AO, Leshchinskaia IB, Biokhimiia. 1981, 46(9):1660-6; Ball TK, Saurugger PN, Benedik MJ, Gene. 1987, 57(2-3):183-92; Biedermann K, Jepsen PK, Riise E, Svendsen I, Carlsberg Res Commun. 1989, 54(1):17-27]. Dieses Enzym wird auch unter dem Markennamen Benzonase vertrieben und im Folgenden "Serratia marcescens Nuklease" genannt.

Um Proteine wirtschaftlich in ausreichenden Mengen und der benötigten Reinheit herstellen zu können, werden sie häufig mittels Standardexpressionsorganismen mittels heterologer Expression hergestellt, d.h. die genetische Information für das gewünschte Protein wird in den Expressionsorganismus eingebracht, der dann die Expression, d.h. Synthese des ihm fremden Proteins übernimmt. Dies hat häufig den Vorteil, dass in diesen Expressionsorganismen die Ausbeute im Vergleich zum Herkunftsorganismus sehr deutlich gesteigert werden kann und etablierte Verfahren zur Kultivierung der Expressionsorganismen und deren weiteren Behandlung zur Produktgewinnung zur Verfügung stehen.

Nukleasen können bei einer gestörten oder fehlerhaften Expression ein großes toxisches Potential auf den Wirtsorganismus ausüben. Falls die Nuklease bereits im Cytosol in eine aktive Form gelangt, würde sie die Nukleinsäuren des Wirtes spalten und zu dessen Absterben oder zur Wachstumshemmung führen. Ebenso kann ein Fehler in der Faltung oder Sekretion dazu führen, dass der Sekretionsapparat des Wirtes blockiert oder beeinträchtigt wird, was ebenso zum Absterben oder zur Wachstumshemmung führen kann.

In EP 229 866 B1 ist die rekombinante Expression der *Serratia marcescens* Nuklease in dem gram-negativen Bakterium *Escherichia coli* beschrieben und in Vergleich zu der Expressionsausbeute im Wildstamm - Serratia marcescens W225 - beschrieben. Auf Seite 13 und Seite 14, Tabelle 3 ist gezeigt, dass mit dem verwendeten System eine Nuklease-Ausbeute von 35 Units / ml Kultur mit dem rekombinanten *E. coli* Stamm und von 7 Units / ml mit dem Wildstamm erreicht wurde. Weiterhin wird offenbart, dass ca. die Hälfte der Aktivität im Periplasma von *E. coli* verbleibt und nicht ins Medium sekretiert wird (Tabelle 4 in EP 229 866 B1).

Biedermann K, Fiedler H, Larsen BS, Riise E, Emborg C, Jepsen PK, Appl Environ Microbiol. 1990, 56(6):1833-8 beschreiben ebenfalls die Sekretion einer *Serratia marcescens* Nuklease (aus dem *Serratia marcescens* Stamm W280) in *E. coli.* Die Studie zeigt einen Vergleich der Sekretionsraten der Nuklease im homologen gram-negativen Wirtsorganismus *Serrratia marcescens* und dem ebenfalls gram-negativen Modellorganismus *E. coli.* In der Veröffentlichung werden Nuklease-Ausbeuten pro ml Kultur unter Fermentationsbedingungen berichtet (Tabelle 1, S. 1837), die 16.500 Units / ml entsprechen.

Ebenfalls finden sich im Stand der Technik Arbeiten zur homologen Expression einer Ribonuklease im gram-positiven Wirt *Bacillus subtilis* (Nakamura A, Koide Y, Miyazaki H, Kitamura A, Masaki H, Beppu T, Uozumi T,.Eur. J. Biochem. 1992, 209(1): 121-127). Es werden Sekretions-Ausbeuten von 7,2 Units / ml berichtet.

Auch ist im Stand der Technik eine Expression einer heterologen Nuklease in einem gram-positiven Bakterium beschrieben (Dieye Y, Usai S., Clier A, Gruss A, Piard J-C, J. Bact. 2001, 183(14): 4157-4166). In dieser Studie wird die Nuklease aus dem gram-positiven Bakterium Staphylococcus aureus in dem gram-positiven Bakterium *Lactobacillus lactis* exprimiert. Ziel dieser Studie ist insbesondere, mittels *L. lactis* ein System zur Expression von gewünschten Proteinen im Darm von Menschen bzw. Tieren zu etablieren (Seite 4157, linke Spalte).

Miyoshi A., et al, FEMS Microbiology Letters 2004, 239: 205-212 beschreiben den Gebrauch eines Xylose-induzierbaren Promoters um cytoplasmische und sekretierte Proteine aus den nuklease Genen von Staph. aureus zu gewinnen.

Die Expression der Nuklease von Staph. aureus in Bacillus subtilis wird von Kovacevic S., et al, J. of Bacteriology 1985, 162 (2): 521-528, beschrieben.

Terpe, K., Applied Microbiology and Biotechnology 2006, 72 (2): 211-222 gibt eine Übersicht über bakterielle Expressionssysteme für heterologe Protein-Produktion.

Die Gram-Färbung ist ein wichtiges Kriterium für die Unterscheidung von Bakterien nach dem Aufbau ihrer Zellwand. Sie beruht auf dem unterschiedlichen Aufbau der Bakterienhülle aus verschiedenen Peptidoglycanen sowie Teichonsäuren. Gram-positive Bakterien besitzen dabei eine dickere, mehrschichtige Mureinhülle, die bis zu 50 % der Hüllentrockenmasse ausmachen kann. Zusätzlich enthält die Zellwand zwischen 20 und 40 % Teichonsäuren. Gramnegative Bakterien dagegen haben nur eine dünne, einschichtige Mureinhülle, die nur etwa 10 % der Trockenmasse der Bakterienhülle ausmacht und keine Teichonsäuren enthält. Methoden zur Durchführung der Gram-Färbung sind dem Fachmann bekannt. Beispiele für gram-negative Bakterien sind alle Arten der Abteilung Proteobacteria wie die Enterobakterien (*Escherichia coli, Salmonella, Shigella, Klebsiella, Proteus, Enterobacter*) oder *Pseudomonas, Legionella, Neisseria. Serratia marcescens,* der Herkunftswirt der *Serratia narcescens* Nuklease, ist ebenfalls ein gram-negatives Bakterium. Beispiele für gram-positive Bakterien sind Actinobacteria und Stämme der Firmicutes (z. B. *Streptococcus, Enterococcus, Staphylococcus, Listeria, Bacillus, Clostridium, Lactobacillus*).

Gram-negative Bakterien im Allgemeinen und *E. coli* im Speziellen zeichnen sich durch einige Nachteile aus. Zum einen ist die Sekretion häufig nur in geringen Ausbeuten möglich und führt meist nur ins Periplasma und nicht direkt ins Medium, was ggf. notwendige nachfolgende Reinigungen erschwert. Zum anderen bilden gram-negative Bakterien häufig im hohen Maße Endotoxine. Sie sind aus einem hydrophilen Polysaccharid- und einem lipophilen Lipidanteil aufgebaut. Im Gegensatz zu den Bakterien, aus denen sie stammen, sind Endotoxine sehr hitzestabil und überstehen sogar die Sterilisation. Endotoxine gehören zu den Pyrogenen, d. h. sie können bei Kontakt mit Schleimhäuten und bei Übertritt ins Blut bei Menschen und manchen Tierarten Fieber erzeugen. Außerdem aktivieren sie eine Reihe von Signalwegen von immunkompetenten Zellen, die entweder zu einer Entzündung oder zu einem programmierten Zelltod (Apoptose) dieser Zellen führen können. Sie sind schon in niedrigsten Konzentrationen (unterer pg/mL-Bereich) biologisch wirksam.

Deswegen sind aufwendige Reinigungsverfahren notwendig, um von Proben, welche direkt oder indirekt in die menschliche bzw. tierische Blutbahn gelangen können, diese Endotoxine unterhalb der biologisch wirksamen Konzentration abzureichern. Besonders relevant ist dieser Sachverhalt für pharmazeutische Anwendungen.

Es besteht somit ein Bedarf an Nukleasen, welche Vorteile gegenüber dem Stand der Technik haben. Insbesondere sollen Nukleasen in wirtschaftlich relevanten Mengen bereitgestellt werden können, sowie ein Verfahren, welches die Nachteile des Standes der Technik hinsichtlich der Gewinnung von Nukleasen verringert oder gar vermeidet.

Diese Aufgabe wird durch den Gegenstand der Patentansprüche gelöst.

Die Erfindung betrifft ein Verfahren zur Herstellung einer Nuklease oder einer Nuklease-Präparation enthaltend eine Nuklease eines gram-negativen Bakteriums umfassend die Expression der Nuklease in einem gram-positiven Bakterium der Gattung *Bacillus* und die anschließende Sekretion der Nuklease, wobei
- die Aminosäuresequenz der Nuklease 60% Homologie mit SEQ ID NO:3 aufweist;
- die Expression mit Hilfe eines induzierbaren Promotors gesteuert wird; der
- durch ein äußeres Signal induziert wird; und/oder
- der Maltose-Promotor einer *Bacillus sp.* ist; und
- ein DNA-Segment, enthaltend eine DNA-Sequenz, welche für die Nuklease codiert, und eine DNA-Sequenz, welche für eine Sekretionssequenz codiert, in das gram-positive Bakterium der Gattung *Bacillus* eingebracht werden; wobei die Sekretionssequenz ausgewählt ist aus den Sekretionssequenzen für
- *amyE* aus *B*. *subtilis* gemäß SEQ ID NO:5 oder
- *B*. *amyloliquefaciens* gemäß SEQ ID NO:170.

Überraschenderweise wurde gefunden, dass sich Nukleasen eines gram-negativen Bakteriums mit hohen Ausbeuten und großer Reinheit in gram-positiven Bakterien mittels heterologer Expression herstellen lassen. Insbesondere wurde gefunden, dass die *Serratia marcescens* Nuklease sich in *Bacillus* sp. effizient durch Sekretion exprimieren lässt.

Die nachfolgende Tabelle fasst die erreichten Ausbeuten an exprimierter Nuklease in unterschiedlichen Wirten zusammen. Bei (1) handelt es sich um die Expression der Nuklease aus dem gram-positiven Wirt *Bacillus subtilis* in dem gram-positiven Wirt *Bacillus subtilis* (Nakamura et al. Eur. J. Biochem. 209, 121 - 127 (1992)), (2) zeigt die beste Expressionsausbeute der Nuklease aus dem gram-negativen Wirt *Serratia marcescens* in dem gram-negativen Wirt *Escherichia coli* (Ausführungsbeispiel 7) und (3) zeigt die Expressionsausbeute der Nuklease aus dem gram-negativen Wirt *Serratia marcescens* in dem gram-positiven Wirt *Bacillus subtilis* (erfindungsgemäßes Verfahren; Plasmid-Konstrukt 5 im Ausführungsbeispiel 6):

| | 1 | 2 | 3 |
|---|---|---|---|
| Nuklease-Herkunft | Gram-positiv | Gram-negativ | Gram-negativ |
| Expressionswirt | Gram-positiv | Gram-negativ | Gram-positiv |
| Ausbeute | 7,2 U/ml | 74 U/ml (Kolben) | 3700 U/ml |
| | | 86 U/ml (Fermenter) | |

Es wird ersichtlich, dass bei dem erfindungsgemäßen Verfahren eine um ein Vielfaches höhere Expressions-Ausbeute erreicht wird.

Dabei ist zu beachten, dass *Bacillus* sp. bereits bei ca. 30 °C das Proliferationsoptimum aufweist, wohingegen beispielsweise *E.coli* ein solches Optimum bei ca. 37 °C aufweist. Daraus ergeben sich u.a. Vorteile im Hinblick auf die bei der Fermentation im großtechnischen Maßstab eingesparte Energiemenge.

Es wurde überraschend gefunden, dass durch das erfindungsgemäße Verfahren Nukleasen bzw. Nuklease-Präparationen erhältlich sind, welche sich durch keine oder allenfalls geringfügige Verunreinigungen durch Endotoxine auszeichnen. Dies bringt besondere Vorteile mit sich, da auf aufwendige Reinigungsverfahren zur Abtrennung von Endotoxinen verzichtet werden kann.

Der Begriff "Endotoxine" wird im Stand der Technik für verschiedene Substanzen verwendet. Neben der im Rahmen der vorliegenden Erfindung verwendeten Bedeutung werden im Stand der Technik vereinzelt auch bestimmte Substanzen von gram-positiven Bakterien als "Endotoxin" bezeichnet, so z.B. das "delta endotoxin" aus *Bacillus thuringiensis* oder bestimmte Substanzen aus *Listeria monocytogenes.*

Im Sinne der vorliegenden Erfindung umfasst der Begriff jedoch bevorzugt ausschließlich Toxine, welche in der äußeren Membran bestimmter gram-negativer Bakterien oder Blaualgen vorkommen und bei denen es sich chemisch um Lipopolysaccharide (LPS) handelt.

Überraschenderweise gelingt die Expression und Sekretion, ohne dass die Nuklease bereits im Cytosol den Produktionsprozess störende Aktivitäten entfaltet oder den Sekretionsapparat des Wirtes in der Art beeinträchtigt, dass es zu Wachstums- oder Produktionshemmungen oder zu erhöhter Zelllyse kommt.

Durch die überraschenderweise erfolgreiche Expression einer Nuklease eines gram-negativen Bakteriums in einem gram-positiven Bakterium ist es nun möglich, die exprimierte und sekretierte Nuklease unmittelbar aus dem Medium zu gewinnen. Eine Lyse von Zellen, wie dieses bei gram-negativen Bakterien, bedingt durch die häufige Unfähigkeit der Sekretion ins Medium, im Regelfall notwendig ist, um die exprimierten Proteine aus dem Periplasma bzw. Cytosol zu isolieren, ist im Rahmen des erfinderischen Verfahrens nicht erforderlich.

Aufgrund der gänzlich anders aufgebauten Zellhülle und dem daraus resultierenden unterschiedlichen Sekretionsmechanismen der gram-positiven Bakterien können ferner weitere Nachteile vermieden werden, die bei der heterologen Expression von Proteinen in gram-negativen Bakterien auftreten können. Hierzu gehört beispielsweise das Auftreten von Einschlußkörpern (*inclusion bodies*) in gram-negativen Zellen, welches u.a. durch eine Überlastung der Sekretionssysteme des gram-negativen Bakteriums durch eine entsprechend hohe Expression verursacht sein kann.

Nukleasen im Sinne der vorliegenden Erfindung sind bevorzugt alle Enzyme, welche den folgenden EC-Klassen der International Union of Biochemistry and Molecular Biology zugeordnet sind: EC 3.1.11, EC 3.1.13, EC 3.1.14, EC 3.1.15, EC 3.1.16, EC 3.1.21, EC 3.1.25, EC 3.1.26, EC 3.1.27, EC 3.1.30, EC 3.1.31.

Nukleasen können DNA oder RNA oder beides spalten. Unter "DNase" wir dabei jegliche DNA spaltende Aktivität, unter "RNase" jegliche RNA spaltende Aktivität verstanden.

In einer bevorzugten Ausführungsform ist die Nuklease eine reine DNase ohne RNase-Aktivität.

In einer anderen bevorzugten Ausführungsform ist die Nuklease eine reine RNase ohne DNase-Aktivität.

In einer weiteren bevorzugten Ausführungsform weist die Nuklease sowohl DNase- als auch RNase-Aktivität auf.

Besonders bevorzugt sind somit Enzyme, welche den EC-Klassen 3.1.21.1, 3.1.21.2, 3.1.21.5, 3.1.22.5, 3.1.26., 3.1.27 zugeordnet sind.

Ganz besonders bevorzugt sind Enzyme, welche der EC-Klasse 3.1.30 und 3.1.31 insbesondere der EC-Klasse 3.1.30.2 zugeordnet sind.

Am bevorzugtesten sind die Enzyme gekennzeichnet durch die Zugangsnummern von Swissprot oder TrEMBL: P13717, A1JRS9, A8GCS3, Q15YD6, Q4HET4, Q0P9S3, B9D1S3, Q4HQP0, Q4HP86, Q4HSK4, B7LWY5, B7LJ21, B7LJB7, B7LIP6, A4G9G4, A7JPX5, Q3JB45, Q3JBS7, A8R779, P29769, Q5LPJ4.

Bevorzugt umfasst der Begriff "Nuklease" ein Protein, welches durch eine Aminosäure-Sequenz gekennzeichnet ist, welche eine Homologie von mindestens 60%, bevorzugt mindestens 70%, besonders bevorzug mindestens 80%, ganz besonders bevorzugt mindestens 90% zu einer nativen Nuklease aufweist und welches außerdem Nuklease-Aktivität aufweist.

"Nuklease-Aktivität" im Sinne der Erfindung bedeutet bevorzugt, dass das Protein DNA oder RNA oder DNA und RNA zu Oligonukleotiden oder Mononukleotiden spalten kann. Bevorzugt erfolgt diese Spaltung in wässrigen Lösungen bei einer Inkubationstemperatur von -20 bis 60 °C und es erfolgt ein Abbau von hochmolekularer, säureunlöslicher DNA und/oder RNA zu niedermolekularen, säurelöslichen Oligo- und/oder Mononukleotiden.

Besonders bevorzugt sind Proteine, welche eine spezifische Nuklease-Aktivität aufweisen, die um 5% höher ist als die spezifische Aktivität der nativen Nuklease, zu welcher sie eine entsprechende Homologie aufweisen. Spezifische Aktivität ist hierbei die katalytische Aktivität einer definierten Proteinmenge der Nuklease. Besonders bevorzugt sind Modifikationen, die vorzugsweise zu mehr als 10%, bevorzugter mehr als 20%, noch bevorzugter mehr als 50% und am bevorzugtesten mehr als 100% Erhöhung der spezifischen Aktivität der Wildtyp-Nuklease führen.

Besonders bevorzugt werden für die Nukleasen codierende DNA-Sequenzen verwendet, die im Hinblick auf das gram-positive Bakterium codon-optimiert sind, was durch die Verwendung optimaler Basentripletts für die entsprechende Aminosäure verwirklicht werden kann.

Im Sinne der Erfindung wird die Homologie einer Sequenz bevorzugt berechnet als Identität mittels BLASTP 2.2.20+ (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402; Stephen F. Altschul, John C. Wootton, E. Michael Gertz, Richa Agarwala, Aleksandr Morgulis, Alejandro A. Schäffer, and Yi-Kuo Yu (2005) ".

Die Nuklease weist eine Homologie von mindestens 60% zu SEQ_ID_3, welche die Aminosäuresequenz der *S. marcescens* Nuklease wiedergibt, auf.

Insgesamt sind Nukleasen untereinander nur wenig konserviert. So weist die oben genannte Nuklease des gram-positiven Bakteriums *Staphylococcus aureus* eine allenfalls geringe Homologie zu der Nuklease des gram-negativen Bakteriums *Serratia marcescens* auf.

Der Begriff "Nuklease eines gram-negativen Bakteriums" im Sinne der vorliegenden Erfindung ist bevorzugt ausschließlich über die Primärsequenz der reifen Nuklease bzw. der für diese Primärsequenz codierende DNA-Sequenz des entsprechenden Wildtyp-Bakteriums definiert. Sollte die Expression im gram-positiven Bakterium zu post-translationalen Modifikationen der Nuklease führen, welche in gram-negativen Bakterien nicht auftreten oder umgekehrt, so handelt es sich im Sinne der Beschreibung dennoch um eine Nuklease eines gram-negativen Bakteriums.

Von dem Begriff "Nuklease eines gram-negativen Bakteriums" im Sinne der vorliegenden Erfindung vorzugsweise nicht eingeschlossen sind Aminosäuresequenzen, die zunächst im Rahmen der Expression des die Nuklease codierenden Genes ebenfalls exprimiert werden, die aber nicht Teil der reifen Nuklease sind. Zu diesen nicht mit eingeschlossenen Aminosäuren gehören beispielsweise Sekretionssequenzen. Sekretionssequenzen sind dem Fachmann auch als Signalpeptide bekannt.

Die Bedeutung des Begriffs "gram-negative Bakterien" im Sinne der vorliegenden Erfindung ist gleichbedeutend mit der Bedeutung des Begriffes im Stand der Technik. Gram-negative Bakterien, welche bevorzugt als Quelle für Nukleasen dienen und somit bevorzugte gram-negative Bakterien im Sinne der vorliegenden Erfindung sind, sind bevorzugt alle Bakterien der Klassen der Proteobacteria wie Alphaproteobacteria, Betaproteobacteria, Gammaproteobacteria, Deltaproteobacteria, Epsilonproteobacteria.

Besonders bevorzugt sind Bakterien aller Ordnungen der Gammaproteobacteria wie Acidithiobacillales, Aeromonadales, Alteromonadales, Cardiobacteriales, Chromatiales, Enterobacteriales, Legionellales, Methylococcales, Oceanospirillales, Pasteurellales, Pseudomonadales, Thiotrichales, Vibrionales, Xanthomonadales.

Bevorzugte Gattungen der Enterobacteriales sind *Arsenophonus, Brenneria, Buchnera, Budvicia, Buttiauxella, Cedecea, Citrobacter, Dickeya, Edwardsiella, Enterobacter, Enwinia, Escherichia, Ewingella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Raoultella, Saccharobacter, Salmonella, Samsonia, Serratia, Shigella, Sodalis, Tatumella, Thorsellia, Trabulsiella, Wigglesworthia, Xenorhabdus, Yersinia, Yokenella.*

Am meisten bevorzugt sind Bakterien der Gattung der *Serratia* wie *S. entomophila, S. ficaria, S. fonticola, S. grimesii, S. liquefaciens, S. odorifera, S. plymuthica, S. proteamaculans, S. quinivorans, S. rubidaea, S. ureilytica.* Am bevorzugsten die Art *Serratia marcescens.*

Die Bedeutung des Begriffs "gram-positive Bakterien" im Sinne der vorliegenden Erfindung ist gleichbedeutend mit der Bedeutung des Begriffes im Stand der Technik.

Beispiele für gram-positive Bakterien sind die Bakterien aller Stämme der Actinobacteria und Firmicutes, z.B. Bakterien der Klassen Bacilli, Clostridia und Mollicutes, z.B. der Familien *Aerococcaceae, Carnobacteriaceae, Enterococcaceae, Lactobacillaceae, Leuconostocaceae, Oscillospiraceae, Streptococcaceae* und die Bacillales mit den Familien *Alicyclobacillaceae, Bacillaceae, Caryophanaceae, Listeriaceae, Paenibacillaceae, Planococcaceae, Sporolactobacillaceae, Staphylococcaceae, Thermoactinomycetaceae, Turicibacteraceae.*

Beispiele sind Bakterien, die zu Gattungen der Familie *Bacillaceae* wie *Alkalibacillus, Amphibacillus, Anoxybacillus, Bacillus, Caldalkalibacillus, Cerasibacillus, Exiguobacterium, Filobacillus, Geobacillus, Gracilibacillus, Halobacillus, Halolactibacillus, Jeotgalibacillus, Lentibacillus, Marinibacillus, Oceanobacillus, Ornithinibacillus, Paraliobacillus, Paucisalibacillus, Pontibacillus, Pontibacillus, Saccharococcus, Salibacillus, Salinibacillus, Tenuibacillus, Thalassobacillus, Ureibacillus, Virgibacillus* gehören.

Bevorzugte Beispiele für Bakterien der Gattung *Bacillus* sind *B. acidiceler, B. acidicola, B. acidocaldarius, B. acidoterrestris, B. aeolius, B. aerius, B. aerophilus, B. agaradhaerens, B. agri, B. aidingensis, B. akibai, B. alcalophilus, B. algicola, B. alginolyticus, B. alkalidiazotrophicus, B. alkalinitrilicus, B. alkalitelluris, B. altitudinis, B. alveayuensis, B. alvei, B. amylolyticus, B. aneurinilyticus, B. aneurinolyticus, B. anthracis, B. aquimaris, B. arenosi, B. arseniciselenatis, B. arsenicoselenatis, B. arsenicus, B. arvi, B. asahii, B. atrophaeus, B. aurantiacus, B. axarquiensis, B. azotofixans, B. azotoformans, B. badius, B. barbaricus, B. bataviensis, B. beijingensis, B. benzoevorans, B. bogoriensis, B. boroniphilus, B. borstelensis, B. butanolivorans, B. carboniphilus, B. cecembensis, B. cellulosilyticus, B. centrosporus, B. chagannorensis, B. chitinolyticus, B. chondroitinus, B. choshinensis, B. cibi, B. circulans, B. clarkii, B. clausii, B. coagulans, B. coahuilensis, B. cohnii, B. curdlanolyticus, B. cycloheptanicus, B. decisifrondis, B. decolorationis, B. dipsosauri, B. drentensis, B. edaphicus, B. ehimensis, B. endophyticus, B. farraginis, B. fastidiosus, B. firmus, B. flexus, B. foraminis, B. fordii, B. formosus, B. fortis, B. fumarioli, B. funiculus, B. fusiformis, B. galactophilus, B. galactosidilyticus, B. gelatini, B. gibsonii, B. ginsengi, B. ginsengihumi, B. globisporus, B. globisporus subsp. globisporus, B. globisporus subsp. marinus, B. glucanolyticus, B. gordonae, B. halmapalus, B. haloalkaliphilus, B. halodenitrificans, B. halodurans, B. halophilus, B. hemicellulosilyticus, B. herbersteinensis, B. horikoshii, B. horti, B. humi, B. hwajinpoensis, B. idriensis, B. indicus, B. infantis, B. infernus, B. insolitus, B. isabeliae, B. jeotgali, B. kaustophilus, B. kobensis, B. koreensis, B. kribbensis, B. krulwichiae, B. laevolacticus, B. larvae, B. laterosporus, B. lautus, B. lehensis, B. lentimorbus, B. lentus, B. litoralis, B. luciferensis, B. macauensis, B. macerans, B. macquariensis, B. macyae, B. malacitensis, B. mannanilyticus, B. marinus, B. marisflavi, B. marismortui, B. massiliensis, B. methanolicus, B. migulanus, B. mojavensis, B. mucilaginosus, B. muralis, B. murimartini, B. mycoides, B. naganoensis, B. nealsonii, B. neidei, B. niabensis, B. niacini, B. novalis, B. odysseyi, B. okhensis, B. okuhidensis, B. oleronius, B. oshimensis, B. pabuli, B. pallidus, B. pallidus (illeg.), B. panaciterrae, B. pantothenticus, B. parabrevis, B. pasteurii, B. patagoniensis, B. peoriae, B. plakortidis, B. pocheonensis, B. polygoni, B. polymyxa, B. popilliae, B. pseudalcaliphilus, B. pseudofirmus, B. pseudomycoides, B. psychrodurans, B. psychrophilus, B. psychrosaccharolyticus, B. psychrotolerans, B. pulvifaciens, B. pycnus, B. qingdaonensis, B. reuszeri, B. ruris, B*. *safensis, B. salarius, B. salexigens, B. saliphilus, B. schlegelii, B. selenatarsenatis, B. selenitireducens, B. seohaeanensis, B. shackletonii, B. silvestris, B. simplex, B. siralis, B. smithii, B. soli, B. sonorensis, B. sphaericus, B. sporothermodurans, B. stearothermophilus, B. stratosphericus, B. subterraneus, B. subtilis subsp. spizizenii, B. subtilis subsp. subtilis, B. taeanensis, B. tequilensis, B. thermantarcticus, B. thermoaerophilus, B. thermoamylovorans, B. thermoantarcticus, B. thermocatenulatus, B. thermocloacae, B. thermodenitrificans, B. thermoglucosidasius, B. thermoleovorans, B. thermoruber, B. thermosphaericus, B. thiaminolyticus, B. thioparans, B. thuringiensis, B. tusciae, B. validus, B. vallismortis, B. vedderi, B. velezensis, B. vietnamensis, B. vireti, B. vulcani, B. wakoensis, B. weihenstephanensis.*

Insbesondere bevorzugt sind die Arten *B. amyloliquefaciens, B. brevis, B. cereus, B. licheniformis, B. megaterium, B. pumilus, B. subtilis.*

Ganz besonders bevorzugt ist somit ein Verfahren zur Herstellung einer Nuklease-Präparation enthaltend eine Nuklease aus *Serratia marcescens* oder, noch bevorzugter, einer Nuklease aus *Serratia marcescens* umfassend die Expression der Nuklease in *Bacillus sp*. und die anschließende Sekretion der Nuklease, bevorzugt unter Verwendung einer heterologen Sekretionssequenz.

Für die Sekretion optimierte Stämme von *B*. *sp* werden erfindungsgemäß bevorzugt eingesetzt. Solche Stämme sind dem Fachmann bekannt. In diesem Zusammenhang wird beispielsweise verwiesen auf WO 99/004019, WO00/039323, WO04/060909.

Der Begriff "Nuklease-Präparation" im Sinne der Erfindung umfasst eine Zusammensetzung enthaltend eine Nuklease, welche nach dem erfindungsgemäßen Verfahren erhältlich ist. Der Begriff umfasst insbesondere auch Zusammensetzungen, welche zusätzlich die erfindungsgemäßen Bakterien oder Bestandteile dieser enthalten, und Zusammensetzungen, welche durch Aufreinigung der erfindungsgemäß hergestellten Protease erhältlich sind.

In einer bevorzugten Ausführungsform ist die Zusammensetzung flüssig und/oder fest und/oder gelartig.

Es wird ein DNA-Segment enthaltend eine DNA-Sequenz, welche für die Nuklease codiert, und eine DNA-Sequenz, welche für eine Sekretionssequenz codiert, in ein gram-positives Bakterium der Gattung *Bacillus* eingebracht.

Erfindungsgemäß wird dazu innerhalb des DNA-Segmentes der DNA-Sequenz, welche für die reife Nuklease codiert, eine weitere DNA-Sequenz vorangestellt, welche für ein Signalpeptid (die Sekretionssequenz) codiert, welches vom Sekretionsapparat des Produktionswirtes erkannt wird und zu einer Sekretion der Nuklease führt.

Gene für Nukleasen aus gram-negativen Bakterien können neben Sequenzen für die reife Nuklease auch für Sekretionssequenzen codieren, welche zu einer Sekretion der Nuklease in das Periplasma der Ursprungsorganismus oder auch das umgebende Medium führt. Beispielsweise verfügt das Gen für die *Serratia narcescens* Nuklease (SEQ-ID 1) über einen Sequenzabschnitt, der für die Sekretionssequenz codiert (SEQ-ID 2) und einen, der für die reife Nuklease codiert (SEQ-ID 3).

In einer bevorzugten Ausführungsform wird in dem erfindungsgemäßen Verfahren eine DNA-Segment in den Wirtsorganismus eingebracht, welche sowohl eine DNA-Sequenz enthält, welche für die native Sekretionssequenz codiert als auch eine DNA-Sequenz enthält, die für die native Proteinsequenz der reifen Nuklease bzw. entsprechend homologen Proteinsequenzen codiert.

Diese native Sekretionssequenz wird als "homolog" bezeichnet.

Der Begriff "homolog" im Sinne der Erfindung bedeutet, dass die Sekretionssequenz identisch ist mit Sequenzen, die in einem einzelnen Wildtyp Bakterium vorliegt und dort gemeinsam mit der entsprechenden Nuklease-Sequenz eine funktionelle Einheit bildet, also in einem Wildtyp Bakterium als ein Molekül mit der Nuklease exprimiert wird.

Im Sinne der Erfindung ist davon der Begriff "Homologie" abzugrenzen, welcher an anderer Stelle der Beschreibung definiert wird.

In einer anderen bevorzugten Ausführungsform wird in den Wirtsorganismus ein DNA-Segment eingebracht, welche eine DNA-Sequenz enthält, die für eine andere, nicht native Sekretionssequenz codiert. Eine solche nicht native, fremde Sekretionssequenz wird als heterolog bezeichnet.

Der Begriff "heterolog" im Sinne der Erfindung bedeutet bevorzugt, insbesondere soweit er sich auf Sekretionssequenzen bezieht, dass die Sekretionssequenz nicht identisch ist mit der nativen Sekretionssequenz der Nuklease.

Heterologe Sekretionssequenzen können natürlich vorkommende Sekretionssequenzen oder künstliche Sekretionssequenzen sein. Natürlich vorkommende Sekretionssequenzen sind solche, welche zur Sekretion von Proteinen in ihren jeweiligen Ursprungsorganismen führen.

In einer weiteren bevorzugten Ausführungsform ist das erfindungsgemäße Verfahren dadurch gekennzeichnet, dass die Sekretionssequenz heterolog ist.

Nicht ausschließend aufgezählt können dies zum Beispiel die Sekretionssequenzen für Proteasen und Peptidasen, Amylasen, Glycoamylasen, Zellulasen, Lipasen, Esterasen, Arabinasen, Glucanasen, Chitosanasen, Lyasen, Xylanasen, Nukleasen, Phosphatasen, Transport- und Bindeproteine, Flagellen oder Phagen/Viren bezogene Proteine in Pro- und Eukaryoten sein

Der Begriff "heterolog" im Sinne der Erfindung bedeutet, dass die Sekretionssequenz nicht identisch ist mit Sequenzen, die in einem einzelnen Wildtyp Bakterium vorliegt und dort gemeinsam mit der entsprechenden Nuklease-Sequenz eine funktionelle Einheit bildet, d.h. eine heterologe Sekretionssequenz kann die Sekretionssequenz eines anderen Proteins des Ursprungsorganismus der Nuklease sein, sie kann eine Sekretionssequenz eines anderen Proteins eines beliebigen anderen Organismus sein oder eine künstliche Sekretionssequenz sein.

Besonders bevorzugt werden heterologe Sekretionssequenzen aus gram-positiven Bakterien verwendet.

Künstliche Sekretionssequenzen sind solche, die nicht in Wildtyp Organismen vorkommen. Aufgrund der Kenntnis des Erkennungsmechanismen und/oder Vergleiche bekannter Sekretionssequenzen sind solche künstlichen Sekretionssequenzen für den Fachmann einfach herstellbar. Aus dem Stand der Technik sind Assays bekannt, mit denen die Eignung einer künstlich hergestellten Sequenz als Sekretionssequenz überprüft werden kann.

Der Begriff "künstliche Sekretionssequenzen" umfasst insbesondere auch Aminosäure - Sequenzen, die eine Homologie von 60%, bevorzugt 70%, besonders bevorzugt 80%, ganz besonders bevorzugt 90% zu den Protein-Sequenzen nativer Sekretionssequenzen aufweisen und, anstatt einer nativen Sekretionssequenz, die Sekretion einer Nuklease in einem gram-positiven Bakterium bewirken.

Besonders bevorzugt sind künstliche Sekretionssequenzen, die zu einer Erhöhung der Sekretionsmenge des Nuklease um mindestens 5%, bevorzugt um mindestens 20%, besonders bevorzugt mindestens 50%, ganz besonders bevorzugt mindestens 100% führen, verglichen mit der Verwendung von natürlich vorkommenden Sekretionssequenzen zur Sekretion der Nuklease.

Besonders bevorzugt wird eine heterologe Sekretionssequenz verwendet, die ausgewählt ist aus der Gruppe der Sekretionssequenzen aus *Bacilli* für die Proteine codiert von den Genen: *abnA, amyE, appA, aprE, bglC, bglS, bpr, csn, dppE, epr, feuA, fhuD, flgB, flgC, flgE, flgK, flhO*, *flhP, fliD, fliK, fliL, ggt, glpQ, hag, htrA, lipA*, *lytD, mntA, mpr, msmE, nprE, nucB, oppA, opuAC, pbpA, pbpB, pbpC, pbpX, pel, pelB, penP, phoA, phoB*, *phoD, phy, pstS, qcrA, rbsB, sacB, tasA, vpr, wapA, wprA, xepA, xkdG, xkdK, xkdM, xlyA, xynA, xynD, ybdN, ybdO*, *ybfO, ybxI, ycdH, yclQ, ydaJ, ydhF, ydhT, yesO, yfiY, yfkN, yflE, yfmC, yfnI, yhcJ, yhcR, yhdW, yheN, yjcM, yjfA, ykwD, ylqB, yncM, ynfF, yoaW, yocH, yodJ, yolA, yolB, ypjP, yqgS, yqgU, yqiX, yqxI, yrpD, yrpE, yrvJ1, yuaB, yurI, yusA, yusW, yvcE, yvfO, yvgO, yvpA, ywaD, yweA, ywoF, ywtD, ywtF, yxeB, yxiA, yxkC.*

Weiterhin besonders bevorzugt wird eine Sekretionssequenz aus der Gruppe der SEQ-ID 4-170 ausgewählt.

Es wird eine heterologe Sekretionssequenz ausgewählt aus den Sekretionssequenzen für *amyE* aus *B. subtilis* (SEQ-ID 5) oder *B. amyloliquefaciens* (SEQ-ID 170).

Erfindungsgemäß wird das Verfahren durchgeführt durch das Einbringen der genetischen Information für eine homologe oder heterologe Sekretionssequenz und eine Nuklease eines gram-negativen Bakteriums in ein gram-positives Bakterium und Sekretion der Nuklease durch den gram-positiven Organismus.

Es ist von Vorteil wenn ein gram-positives Bakterium zur Sekretion der Nuklease eines gram-negativen Bakteriums ausgewählt wird, welches sich durch ein niedriges Level an sekretierten Proteasen auszeichnet. Ein solches gram-positives Bakterium wird als protease-arm bezeichnet.

In einer bevorzugten Ausführungsform ist das gram-positive Bakterium ein protease-armer Wirt und/oder *Bacillus sp.*

Der Begriff "protease-arm" im Sinne der vorliegenden Erfindung bedeutet dass es sich um eine Art oder ein natürliches Isolat einer Gattung von Bakterien handelt, welche weniger als 50%, bevorzugter weniger als 25%, am bevorzugtesten weniger als 10% des durchschnittlichen Protease-Levels einer anderen Art oder eines natürlichen Isolats der gleichen Gattung aufweist.

Ebenfalls besonders bevorzugt wird ein gram-positives Bakterium zur Sekretion der Nuklease eines gram-negativen Bakteriums ausgewählt, dessen Protease-Level durch künstliche Modifikationen reduziert wurde.

Bevorzugt werden diese Modifikationen zufällig in das ausgewählte gram-positive Bakterium eingebracht. Methoden zur zufälligen Mutationen wie chemische, UV-induzierte, strahlungsinduzierte Mutagenese oder ähnliche Methoden sind dem Fachmann bekannt. Nach Durchführung der Mutagenese werden die erhaltenen Klone nach Klonen mit erniedrigtem Protease-Level durchmustert und die erhaltenen Klone zur Sekretion der Nuklease eingesetzt.

Besonders bevorzugt werden diese Modifikationen zielgerichtet in das ausgewählte gram-positive Bakterium eingebracht. Dabei werden die Gene, welche für sekretierte Proteasen codieren, identifiziert und vollständig oder teilweise derart ersetzt oder modifiziert, dass die entsprechende Protease nicht mehr oder mit verminderten Level sekretiert wird. Methoden zur Modifikation genomischer Sequenzen sind dem Fachmann bekannt. Am bevorzugtesten werden mehrere oder viele der für sekretierte Proteasen codierenden Gene identifiziert und vollständig oder teilweise derart ersetzt oder modifiziert, dass die entsprechende Proteasen nicht mehr oder mit verminderten Level sekretiert werden.

Besonders bevorzugt wird ein gram-positives Bakterium zur Sekretion der Nuklease eines gram-negativen Bakteriums ausgewählt, dessen neutrale Proteasen (z. B. *npr*) und/oder alkalische Proteasen (z.B. *apr*) und/oder weitere Proteasen (z.B. *epr, bpr, mpr*) ganz oder teilweise deletiert ist.

Am bevorzugtesten wird ein *B. amyloliquefaciens, B. brevis, B. cereus, B. licheniformis, B. megaterium, B. pumilus oder B. subtilis -* Stamm, zur Sekretion der Nuklease eines gram-negativen Bakteriums ausgewählt, dessen Protease(n) *npr* und/oder *apr* und/oder *epr* und/oder *bpr* und/oder *mpr* ganz oder teilweise deletiert ist.

Die Expression von Genen wird durch Promotoren gesteuert. Promotoren sind dabei solche DNA-Sequenzen, welche als Erkennungsstellen für die RNA-Polymerasen dienen. Promotoren können neben der eigentlichen Bindungsstelle für die RNA-Polymerase weitere Sequenzen für die Bindung von zusätzlichen aktivierenden oder inhibierenden Faktoren aufweisen oder aber auch Sequenzen, welche durch Bildung von Sekundärstrukturen den Promotor beeinflussen.

Bei Promotoren werden konstitutive und induzierbare Promotoren unterschieden. Beide Arten von Promotoren können erfindungsgemäß eingesetzt werden. Während konstitutive Promotoren permanent abgelesen werden, werden induzierbare Promotoren durch ein zusätzliches Signal im Laufe des Produktionsprozesses eingeschaltet. Dabei kann das Signal durch innere oder äußere Faktoren generiert werden. Ein innerer Faktor wäre zum Beispiel das Erreichen einer bestimmten Wachstumsphase des Organismus, wie der Übergang von der logarithmischen in die stationäre Phase von Bacilli. Ein äußerer Faktor wäre zum Beispiel die Verfügbarkeit einer bestimmten Substrat-Quelle wie zum Beispiel Stärke oder Proteinen oder das Vorhandensein eines definierten Induktors wie zum Beispiel Tetracyclin, Maltose oder IPTG (Isopropyl-[beta]-D-thiogalactopyranosid). Äußere und innere Faktoren können auch inhibierend wirken, das heißt, dass unter bestimmten Bedingungen bzw. dem Vorliegen einer bestimmten Substanz die Ablesung des Promotors unterdrückt wird.

Für die heterologe Expression von Proteinen im Allgemeinen und potentiell toxischen Enzymen wie Nukleasen im Speziellen sind solche Promotoren besonders geeignet, welche während einer bestimmten Phase, der frühen Wachstumsphase der Kultur, unterdrückt bis gar nicht aktiv sind und in einer anderen Phase, der Produktionsphase der Kultur, zu einer starken Expression angeschaltet werden können.

In einer bevorzugten Ausführungsform wird für das erfindungsgemäße Verfahren ein nativer Promotor aus einem gram-positiven Bakterium verwendet, d.h. ein Promotor eines gram-positiven Wildtyp Bakteriums.

In einer bevorzugten Ausführungsform wird die Expression mit Hilfe eines konstitutiven Promotors gesteuert. Ein Beispiel für einen solchen Promotor ist der ß-Glucanase-Promotor von *Bacillus amyloliquefaciens.*

Die Expression wird mit Hilfe eines induzierbaren Promotors gesteuert, der bevorzugt unter nicht induzierten Bedingungen höchstens 30%, bevorzugt höchstens 10%, besonders bevorzugt höchstens 5%, ganz besonders bevorzugt höchstens 1% seiner maximalen Expressionsleistung zeigt.

In einer bevorzugten Ausführungsform kann ein solcher induzierbarer Promotor ein Wachstumsphasen-abhängiger Promotor sein.

Besonders bevorzugt ist hierbei ein Promotor ausgewählt aus der Gruppe der Promotoren für die Gene *abnA, amyE, appA, aprE, bglC, bglS, bpr, csn, dppE, epr, feuA, fhuD, flgB, flgC, flgE, flgK, flhO*, *flhP, fliD, fliK, fliL, ggt, glpQ, hag, htrA, lipA, lytD, mntA, mpr, msmE, nucB, oppA, opuAC, pbpA, pbpB, pbpC, pbpX, pel, pelB, penP, phoA, phoB*, *phoD, phy, pstS, qcrA, rbsB*, *sacB, tasA, vpr, wapA, wprA, xepA, xkdG, xkdK, xkdM, xlyA, xynA, xynD, ybdN, ybdO*, *ybfO*, *ybxI, ycdH, yclQ, ydaJ, ydhF, ydhT, yesO, yfiY, yfkN, yflE, yfmC, yfnI, yhcJ, yhcR, yhdW, yheN, yjcM, yjfA, ykwD, ylqB*, *yncM, ynfF, yoaW, yocH, yodJ, yolA, yolB, ypjP, yqgS, yqgU, yqiX, yqxl, yrpD, yrpE, yrvJ1, yuaB, yurI, yusA, yusW, yvcE, yvfO*, *yvgO, yvpA, ywaD, yweA, ywoF, ywtD, ywtF, yxeB, yxiA, yxkC* aus Bacilli.

Ganz besonders bevorzugt wird ein Promotor für *npr* aus *Bacillus* sp. verwendet.

Der induzierbare Promotor wird durch ein äußeres Signal induziert.

Bevorzugt wird dabei ein Hitze-induzierbarer, ein Sucrose-induzierbarer, ein Stärke-induzierbarer, ein DNA Schäden-induzierbarer, ein Stress-induzierbarer, Antibiotikainduzierbarer, Kälte-induzierbarer, Xylose-induzierbarer, IPTG-induzierbarer, Arabinoseinduzierbarer, Alkali-induzierbarer, Säure-induzierbarer, Inositol-induzierbarer Promotor aus Bacilli verwendet.

Es wird ein Maltose-induzierbarer Promotor aus *Bacillus sp.,* bevorzugt der Maltose-Promotor aus *B. subtilis* (SEQ-ID 171) oder der Maltose-Promotor aus *B. amyloliquefaciens* (SEQ-ID 172) verwendet.

In einer anderen Ausführungsform wird ein Promotor verwendet, der eine Variante eines in dem erfindungsgemäßen Verfahren verwendeten nativen Promotors ist. Ein solcher Promotor wird im Rahmen der Erfindung als "Promotorvariante" bezeichnet.

Promotorvarianten im Sinne der Erfindung sind Varianten, die eine Homologie von mindestens 50 %, bevorzugt mindestens 60%, noch bevorzugter mindestens 70%, besonders, bevorzugt mindestens 80%, ganz besonders bevorzugt mindestens 90% zu einem erfindungsgemäß verwendeten nativen Promotor aufweisen und deren Expressionsleistung mindestens 20%, bevorzugt mindestens 50%, besonders bevorzugt mindestens 100%, noch bevorzugter mindestens 200%, ganz besonders bevorzugt mindestens 300% höher ist als die Expressionsleistung diese nativen Promotors.

Für eine Vielzahl von Anwendungen von Proteinen und Enzymen im Allgemeinen und Nukleasen im Speziellen ist es vorteilhaft, wenn die Proteine, Enzyme und Nukleasen in hochreiner Form vorliegen. Neben klassischen Verfahren der Proteinreinigung, die dem Fachmann bekannt sind, kann die Reinigung der Proteine vereinfacht werden, wenn dem Zielprotein eine zusätzliche Aminosäure-Sequenz (Affinitäts-Tag) angehängt wird, welche eine Interaktion mit einem spezifischen Material erlaubt und somit das Zielprotein an dieses Material bindet und Verunreinigungen oder Nebenprodukte durch Waschen entfernt werden können. Weiterhin besonders vorteilhaft ist dann, dass die angehangene Aminosäure-Sequenz dazu genutzt werden kann, um das Zielprotein dauerhaft an einen Träger zu immobilisieren und es dann dadurch später leicht aus der Anwendung wieder entfernen zu können.

In einer bevorzugten Ausführungsform weist die Nuklease zusätzlich einen Affinitäts-Tag auf.

Somit entsteht ein Fusionsprotein aus Sekretionssequenz, reifer Nuklease und Affinitäts-Tag.

In einer Ausführungsform wird die DNA-Sequenz, welche für das Affinitäts-Tag codiert, an das 3'-Ende der für die Nuklease codierenden DNA-Sequenz angehängt, so dass das Affinitäts-Tag an das C-terminale Ende der reifen Nuklease fusioniert wird.

In einer anderen Ausführungsform wird die DNA-Sequenz, welche für das Affinitäts-Tag codiert, zwischen die DNA-Sequenz welche für die Sekretionssequenz codiert, und das 5'-Ende der für die Nuklease codierenden DNA-Sequenz angehängt, so dass das Affinitäts-Tag an das N-terminale Ende der reifen Nuklease fusioniert wird.

In einer bevorzugten Ausführungsform ist zwischen dem Affinitäts-Tag und der reifen Nuklease bzw. zwischen der reifen Nuklease und dem Affinitätstag ein Abstandshalter aus Aminosäureresten eingefügt. Bevorzugt ist ein Abstandhalter enthaltend nicht mehr als 1000 Aminosäureresten, bevorzugter nicht mehr als 100 Aminosäureresten, noch bevorzugter nicht mehr als 20 Aminosäureresten, besonders bevorzugt nicht mehr als 10 Aminosäureresten, ganz besonders bevorzugt nicht mehr als 5 Aminosäureresten.

Vorzugsweise enthält der Abstandhalter die Erkennungssequenz einer spezifischen Protease, um den Affinitäts-Tag und den Abstandshalter selbst oder Teile des Abstandshalters wieder abspalten zu können.

In einer besonders bevorzugten Ausführungsform wird der Affinitäts-Tag aus der Gruppe der zweiwertige Ionen bindenden Aminosäure-Sequenzen (z. B. His-Tag), Kohlenhydrat- oder Chitin-bindenden Aminosäure-Sequenzen (z.B. Maltose-Bindungsprotein, Zellulose-Bindeprotein oder Chitin-Bindungsprotein) oder Streptavidin-bindenden Aminosäure-Sequenzen (z.B. Strep-Tag) ausgewählt.

Die DNA-Sequenz, welche die Nuklease codiert, kann im Bakterium auf verschiedene Art und Weise vorliegen.

In einer bevorzugten Ausführungsform ist das DNA-Segment enthaltend diese DNA-Sequenz in einen Expressionsvektor integriert.

Vorzugsweise erfolgt die Expression mit Hilfe eines Expressionsvektors. Verschiedene solcher Expressionsvektoren sind dem Fachmann bekannt. Der Begriff "Vektor" im Sinne der Beschreibung umfasst Plasmide, Bakteriophagen, BACs (bacterial artificial chromosomes) und Cosmide.

In einer bevorzugten Ausführungsform des Verfahrens umfasst der Expressionsvektor als Selektionsmarker ein Gen, welches eine Antibiotika-Resistenz codiert. Dieses Resistenz-Gen wird bevorzugt ausgewählt aus der Gruppe bestehend aus Resistenzgenen gegen Kanamycin, Erythromycin, Tetracyclin, Spectinomycin, Chloramphenicol, Streptomycin, Neomycin.

In einer weiteren bevorzugten Ausführungsform des Verfahrens umfasst der Expressionsvektor als Selektionsmarker ein Gen, welches eine Auxotrophie des Bakteriums kompensiert. Als auxotroph werden Wirtsorganismen verstanden, die bestimmte essentielle Substanzen nicht selbstständig synthetisieren können und zur Komplementierung mindestens eine weitere DNA-Sequenz, den Selektionsmarker, benötigen.

In einer ganz besonders bevorzugten Ausführungsform umfasst der Expressionsvektor als Selektionsmarker eine oder mehrere Kopien des glyA-Genes und wird in einem Wirtsorganismus verwendet, bei dem das glyA-Gen deletiert oder zu einer verminderten Expressionsleistung modifiziert ist.

Vorzugsweise erfolgt die Expression der Nuklease mit Hilfe eines Plasmids.

Als Plasmide werden autonom replizierende DNA-Moleküle verstanden, welche extrachromosomal vorliegen und nicht zum Bakterienchromosom gehören.

Bevorzugt hat das Plasmid eine Größe von 2 bis 500 kbp, bevorzugter von 3 bis 100 kbp, noch bevorzugter von 4 bis 20 kbp, ganz besonders bevorzugt von 5 bis 10 kbp.

In einer bevorzugten Ausführungsform des Verfahrens liegt das Plasmid in der Wirtzelle in mehr als einer Kopie, bevorzugter in mehr als 5 Kopien, noch bevorzugter in mehr als 10 Kopien, besonders bevorzugt in mehr als 20 Kopien vor.

Bevorzugt wird ein Replikon des Plasmides gewählt, welches nach 100 Generationen Kultivierung ohne Selektionsdruck noch das Vorliegen des Plasmides in mindestens 20 %, bevorzugter mindestens 50 %, noch bevorzugter mindestens 70 %, besonders bevorzugt mindestens 90 % der Bakterienzellen sicherstellt.

In einer anderen bevorzugten Ausführungsform ist das DNA-Segment enthaltend die DNA-Sequenz zur Expression der Nuklease in das Bakterienchromosom integriert.

Bevorzugt wird mindestens 1 Kopie des DNA-Segments enthaltend diese DNA-Sequenz, bevorzugter mindestens 3 Kopien, noch bevorzugter mindestens 5 Kopien, besonders bevorzugt mindestens 10 Kopien der dieses DNA-Segments in das Bakterienchromosom integriert.

In einer bevorzugten Ausführungsform des Verfahrens wird zusammen mit der DNA-Sequenz, welche die Nuklease codiert, als Selektionsmarker ein Gen in das Bakterienchromosom integriert, welches eine Antibiotika-Resistenz codiert. Dieses Resistenz-Gen wird bevorzugt ausgewählt aus der Gruppe bestehend aus Resistenzgenen gegen Kanamycin, Erythromycin, Tetracyclin, Spectinomycin, Chloramphenicol, Streptomycin, Neomycin.

In einer weiteren bevorzugten Ausführungsform des Verfahrens wird zusammen mit der DNA-Sequenz, welche die Nuklease codiert, als Selektionsmarker ein Gen in das Bakterienchromosom integriert, welches eine Auxotrophie des Bakteriums kompensiert. Als auxotroph werden Wirtsorganismen verstanden, die bestimmte essentielle Substanzen nicht selbstständig synthetisieren können und zur Komplementierung mindestens eine weitere DNA-Sequenz, den Selektionsmarker, benötigen. Dieser Selektionsmarker ist bevorzugt glyA.

In einer weiteren bevorzugten Ausführungsform des Verfahrens wird gar kein Selektionsmarker auf dem Expressionsvektor oder dem integrierten DNA-Segment enthaltend die DNA-Sequenz, welche die Nuklease codiert, verwendet oder der zuvor verwendete Selektionsmarker nachträglich wieder entfernt, so dass ein Selektionsmarker-freier Wirtsstamm entsteht.Weiterhin offenbart ist ein gram-positives Bakterium enthaltend eine Nuklease eines gram-negativen Bakteriums und/oder eine DNA-Sequenz, welche für eine Nuklease eines gram-negativen Bakteriums codiert.

In einer bevorzugten Ausführungsform ist diese DNA-Sequenz in ein Expressionsvektor, vorzugsweise ein Plasmid integriert.

In einer anderen bevorzugten Ausführungsform ist die DNA-Sequenz in das bakterielle Chromosom integriert.

In einer bevorzugten Ausführungsform des Verfahrens sind alle Medienbestandteile des gram-positiven Wirts zur Expression der Nuklease eines gram-negativen Bakteriums aus nicht-tierischen Quellen gewählt.

In einer bevorzugten Ausführungsform des Verfahrens wird der gram-positive Wirt zur Expression der Nuklease eines gram-negativen Bakteriums unter Anwendung eines Fedbatch-Protokolls kultiviert. Unter Fedbatch wird dabei verstanden, dass ein Teil der Nährstoffe zu Beginn der Kultivierung bereits vorliegt und ein weiterer Teil der Nährstoffe ab einem bestimmten Zeitpunkt kontinuierlich oder diskontinuierlich zugeführt wird.

In einer weiteren bevorzugten Ausführungsform wird im Fedbatch-Protokoll eine Kohlenstoffquelle, eine Stickstoffquelle, und eine Phosphatquelle sowie Mischungen aus benötigten Salzen und Spurenelementen und ggf. essentielle Aminosäuren und Selektionsmarker vorgelegt.

In einer bevorzugten Ausführungsform des Verfahrens wird der gram-positive Wirt zur Expression der Nuklease eines gram-negativen Bakteriums unter Anwendung eines Batch-Protokolls kultiviert. Unter Batch wird dabei verstanden, dass alle Nährstoffe zu Beginn der Kultivierung bereits vorliegen und keine weiteren Nährstoffe während der Kultivierung zugeführt werden. Nicht als Nährstoffe gelten Lösungen zur Korrektur von pH-Wert oder Schaumbildung, wie Säuren, Laugen oder Antischaummittel.

In einer weiteren bevorzugten Ausführungsform des Verfahrens wird die Kohlenstoffquelle bevorzugt in einer Konzentration von mehr als 1% Gewichtsanteil pro Volumen, bevorzugter mehr als 3 %, noch bevorzugter mehr als 6 %, besonders bevorzugt mehr als 9 % zugegeben.

In einer bevorzugten Ausführungsform des Verfahrens wird als Kohlenstoffquelle Glukose verwendet.

In einer bevorzugten Ausführungsform des Verfahrens wird als Kohlenstoffquelle ein Dextrin verwendet.

In einer bevorzugten Ausführungsform des Verfahrens wird als Kohlenstoffquelle Maltose verwendet.

In einer weiteren bevorzugten Ausführungsform des Verfahrens wird die Kohlenstoffquelle innerhalb der Kultivierung auf Maltose oder einer Mischung aus Maltose und einer anderen Kohlenstoffquelle umgestellt.

In einer weiteren bevorzugten Ausführungsform des Verfahrens wird hydrolysiertes Pepton, besonders bevorzugt hydrolysiertes Soja-Pepton der Kultivierung zugefügt.

In einer weiteren bevorzugten Ausführungsform des Verfahrens wird TRIS (Tris(hydroxymethyl)-aminomethan) dem Kultivierungsmedium zugefügt.

Das erfindungsgemäße Verfahren führt zu einer hohen Ausbeute an Nuklease.

In einer bevorzugten Ausführungsform des erfindungsgemäßen Verfahrens wird eine Ausbeute von mindestens 5.000, noch bevorzugter mindestens 15.000, noch bevorzugter mindestens 25.000, besonders bevorzugt mindestens 50.000, ganz besonders bevorzugt mindestens 100.000 Nuklease-Units pro ml Kulturvolumen erzielt.

Aufgrund des bestehenden Bedarfes an Endotoxin-freien Nukleasen sind bisher beträchtliche Anstrengungen unternommen worden, Reinigungsschritte zu entwickeln, welcher der Abtrennung von Endotoxinen von Nukleasen oder Nukleasen-Präparationen dienen.

Einen Überblick über verschiedene Methoden zur Entfernung von Endotoxinen, auf denen ein solcher Reinigungsschritte basieren kann, ist zu finden in Magalhaes et al. (2007) J. Pharm. Pharmaceut. Sci 10: 388-404.

Aus dem Stand der Technik bekannte Reinigungsschritte zur Abtrennung von Endotoxinen basieren beispielsweise auf folgenden Methoden: Anionenaustauschchromatographie; Affinititätschromatographie; lonenaustausch-chromatographie, insbesondere lonenaustauschchromatographie unter Verwendung von Alkandiol; Ultrafiltration; Aufreinigung mittels Affinitätsabsorbentien wie beispielsweise L-Histidin, poly-L-Histidin, poly(gamma-methyl L-Glutamat), Polymyxin B; Gelfiltration; Gelfiltrationschromatographie; Sucrose-Gradienten-Zentrifugation; Aufreinigung mittels eines zweiphasigen Mizellen-Systems; Triton X-114-basierte Phasenseparation; Temperatur-induzierte Phasen-Separation; Aufreinigung durch nicht-selektive Adsorption mit hydrophoben Adsorbentien oder Anionen-Austauscher; Polyacrylamidgel-Elektrophorese, inbesondere Slab-Polyacrylamidgel-Elektrophorese; SDS-Gel-Elektrophorese; Membran-basierte Chromatographie; Agarose-Gel-Elektrophorese; Cäsiumchlorid-Gradientenzentrifugation; Affinitäts-Aufreinigungen unter Verwendung von beads.

Die Anwendung dieser Verfahren in der Praxis ist teilweise mit einem signifikanten Aufwand und/oder mit verschiedenen technischen Schwierigkeiten verbunden.

Da das erfindungsgemäße Verfahren auf einer Verwendung von gram-positiven, und nicht auf gram-negativen Bakterien beruht, kann eine Kontamination des Verfahrensproduktes durch Endotoxine vermieden werden.

In einer weiteren bevorzugten Ausführungsform umfasst das erfindungsgemäße Verfahren keinen Reinigungsschritt, welcher der gezielten Abtrennung von Endotoxinen dient.

Umfasst sind somit insbesondere erfindungsgemäße Verfahren, die keinen solchen Reinigungsschritt enthalten, die auf den Methoden basieren, die oben genannt sind oder in den oben angeführten Dokumenten zum Zwecke der Endotoxin-Entfernung beschrieben sind oder daraus abgeleitet sind.

Das erfindungsgemäße Verfahren kann jedoch durchaus Reinigungsschritte umfassen, welche für Enzympräparationen üblich sind. Bevorzugt sind diese Reinigungsschritte jedoch nicht auf die gezielte Abtrennung von Endotoxinen ausgerichtet.Hierin auch offenbart ist eine Nuklease, die durch das erfindungsgemäße Verfahren erhältlich ist.Ebenfalls offenbart ist eine Nuklease-Präparation, die durch das erfindungsgemäße Verfahren erhältlich ist, insbesondere durch die bevorzugte Ausführungsform des erfindungsgemäßen Verfahrens, die keinen Reinigungsschritt umfasst, welcher der gezielten Abtrennung von Endotoxinen dient.

Die Nuklease-Präparation enthält die Nuklease und ggf. andere Inhaltsstoffe. So kann die Nuklease-Präparation insbesondere gram-positive Bakterien oder Komponenten oder Bestandteile von gram-positiven Bakterien umfassen, welche im Zuge der Reinigung der Nuklease mit Hilfe herkömmlicher Reinigungsschritte in der Präparation verbleiben. Diese Komponenten oder Bestandteile können Kohlenhydrate, Fette, Nukleinsäuren oder Proteine sein, bzw. Teile oder Bruchstücke dieser Moleküle. Ebenfalls können die Bestandteile aus charakteristischen Metaboliten oder Sekundär-Metabolite bestehen oder aus diesen abgeleitet sein. Die Komponenten und Bestandteile können üblicherweise mit dem Fachmann bekannten Methoden identifiziert werden. So können Nukleinsäuren zum Beispiel über PCR amplifiziert und durch Sequenzierung identifiziert werden. Stoffwechsel-Metabolite können zum Beispiel über HPLC oder GC-analytische Methoden bestimmt werden. Weitere Methoden zur Identifikation von gram-positiven Zell-Bestandteilen oder -Trümmern sind die Massenspektroskopie, sowie IR-, NMR- oder UV/VIS-Spektroskopie.

Der Begriff "Bestandteil von gram-postiven Bakterien" bzw. "Bestandteile eines gram-positiven Bakteriums" im Sinne der Erfindung beschreibt bevorzugt Metabolite oder sekundäre Metabolite der gram-positiven Bakterien.

Der Begriff "Komponenten von gram-positiven Bakterien" bzw. "Komponenten eines gram-positiven Bakteriums" im Sinne der Erfindung beschreibt bevorzugt Moleküle, die im nativen Genom des Bakteriums kodiert werden sowie Teile des nativen Genoms selber, es umfasst somit Proteine, Peptide, RNA- und DNA-Moleküle

Bevorzugt enhält die Zusammensetzung Komponenten eines gram-positiven Bakteriums.

Der molekulare Anteil der Komponenten von gram-positiven Bakterien kann mehr als 10⁻¹⁰ mol.-% Gesamtmenge der Zusammensetzung, bevorzugter mehr als 10⁻⁸ mol.-% Gesamtmenge der Zusammensetzung, noch bevorzugter mehr als 10⁻⁶ mol.-% Gesamtmenge der Zusammensetzung, am bevorzugtestens mehr als 10⁻⁴ mol.-% Gesamtmenge der Zusammensetzung betragen.

Die Bestandteile und/oder Komponenten des gram-positiven Bakteriums stammen mindestens teilweise von dem/den gram-positiven Bakterien, die zur Expression der Nuklease verwendet wurden.

Die Nuklease-Präparation kann fest sein, beispielsweise ein lyophilisiertes Pulver, pastös oder flüssig sein, beispielseise eine wässrige Lösung oder Dispersion.

Bevorzugt weist eine solche Nuklease-Präparation weniger als 250 Endotoxin-Units (EU) pro Megaunit (MU) Nukleaseaktivität, bevorzugter weniger als 125 Endotoxin-Units pro MU Nukleaseaktivität, noch bevorzugter weniger als 25 Endotoxin-Units pro MU Nukleaseaktivität, besonders bevorzugt weniger als 5 Endotoxin-Units pro MU Nukleaseaktivität, ganz besonders bevorzugt weniger als 1 Endotoxin-Units pro MU Nukleaseaktivität auf.

Im Sinne der vorliegenden Erfindung wird unter 1 Unit Nuklease-Aktivität die Enzymmenge definiert, welche aus hochmolekularer DNA oder RNA in 1 Stunde bei 37°C säurelösliche Oligonukleo-tide freisetzt, welche einer Absorption bei 260 nm von 1 Absorptionseinheit entspricht.

Die Abkürzung U entspricht einer Unit, die Abkürzung kU steht für eintausend Units und die Abkürzung MU steht für eine Mega-Unit, also eine Million Units.

Im Sinne der vorliegenden Erfindung werden Endotoxin-Units (EU) mittels des 1997 im Bereich des Arbeitsschutzes das BIA-Merkblatt 9450 eingeführten chromogen-kinetische Limulustest bestimmt. Als Faktor zum Vergleich zwischen der Konzentrationsangabe in EU und ng wird hier angegeben: 10 EU = 1 ng Endotoxin.

Der Limulus-Amöbozyten-Lysat-Test (LAL-Test; erstmals beschrieben von Bang 1956) ist ein sensitiver und standardisierbar Test zur Bestimmung der Endotoxin-Konzentration. Beim LAL-Test macht man sich die Tatsache zu Nutze, dass die Hämolymphe des Limulus polyphemus (Pfeilschwanzkrebs) in der Gegenwart von Endotoxin koaguliert. Als Vergleichswert gilt ein chemisch reines Standard-Lipopolysaccharid. Bezüglich Einzelheiten wird in diesem Zusammenhang auf das Europäische Arzneibuch verwiesen (z.B. Eur. Ph. 5.0, 2.6.14 "Bacterial Endotoxins"). Vorzugsweise erfolgt der Test in Einklang mit den darin beschriebenen Standardbedingungen.

Ganz besonders bevorzugt ist die Nuklease-Präparation frei von Endotoxinen, d.h. es sind keine Endotoxine mit den vorstehend beschreibenen Methoden nachweisbar.

Da gram-positive Bakterien sekretierte Proteine üblicherweise direkt ins Medium sekretieren (und nicht beispielsweise ins Periplasma) kann bei der Gewinnung der Nuklease ggf. auf einen Schritt verzichtet werden, welcher der Lyse der Bakterien dient.

Die Nuklease wird hierbei bevorzugt direkt aus dem Medium gewonnen. Dem Fachmann sind hierzu verschiedene geeignete Verfahren bekannt. Hierzu gehören beispielsweise Verfahren basierend auf Zentrifugationen/Separationen, Fällungen, Chromatographie-Prozessen und/oder Filtrationen.

Bevorzugt sind somit erfindungsgemäße Verfahren, welche dadurch gekennzeichnet sind, dass sie keinen Schritt umfassen, der einer Lyse der Bakterien dient.

Bevorzugt ist das erfindungsgemäße Verfahren dadurch gekennzeichnet, dass die gram-positiven Bakterien mittels Zentrifugation und/oder Filtration aus dem Medium entfernt werden.

Schritte, die einer Lyse von Bakterien dienen umfassen beispielsweise UV-Beschallung, mechanische Lyse wie beispielsweise Lyse unter Verwendung einer French Press oder eines Manton-Gaulin-Homogenisators, osmotische Lyse, chemische Lyse wie beispielsweise mitttels Zugabe von Lysozym und/oder EDTA und/oder Triton und/oder anderen Detergentien.

Ein weiterer Aspekt betrifft eine Zusammensetzung enthaltend eine Nuklease eines gram-negativen Bakteriums und ein gram-positives Bakterium oder dessen Fragmente oder Bestandteile oder deren Fragmente.

Ein weiterer Aspekt betrifft ein Verfahren zur Hydrolyse von DNA und oder RNA umfassend den Schritt des Zusammenbringens einer Nuklease oder Nuklease-Präparation und der zu hydrolysierenden DNA und/oder RNA unter geeigneten Bedingungen.

Ein weiterer Aspekt betrifft ein gram-positives Bakterium enthaltend
- eine Nuklease eines gram-negativen Bakteriums und/oder
- ein DNA-Sequenz, welche für eine Nuklease eines gram-negativen Bakteriums codiert.

Ein weiterer Aspekt umfasst die Verwendung einer mittels des erfindungsgemäßen Verfahrens erhältlichen Nuklease und/oder Nuklease-Präparation zur Spaltung von DNA und/oder RNA.

Insbesondere hierin beschrieben ist die Verwendung der mittels des erfindungsgemäßen Verfahrens erhältlichen Nuklease und/oder Nuklease-Präparation für die Herstellung von Produkten aus den Bereichen Pharmazie, Kosmetik, Diagnostik, Lebensmitteltechnologie, Biotechnologie.

Produkte aus dem Bereich Pharmazie umfassen insbesondere chemische Wirkstoffmoleküle, biologische Wirkstoffe, insbesondere pharmakologisch wirksame Biomoleküle und pharmazeutische Zusatzstoffe.

Biologische Wirkstoffe sind beispielsweise Antikörper, Antikörperfragmente, Proteine, Peptide, genetisch veränderte und/oder inaktivierte Viren oder Virenpartikel und Nukleinsäuren.

Produkte aus dem Bereich Kosmetik umfassen insbesondere Proteine, Peptide, biologische Wirkstoffe und kosmetische Zusatzstoffe.

Produkte aus dem Bereich Diagnostik umfassen insbesondere Proteine, Peptide, Enzyme, Antikörper, Antikörperfragmente, Antigene, Nukleinsäuren, Enzym- Substrate, Cofaktoren und Zusatzstoffe.

Produkte aus dem Bereich Lebensmitteltechnologie umfassen insbesondere Proteine, Enzyme, Nährstoffe, Nahrungsmittelergänzungsstoffe, Zusatzstoffe wie Konservierungsstoffe, Farbstoffe.

Produkte aus dem Bereich Biotechnologie umfassen insbesondere Proteine, Enzyme und Nukleinsäuren, Chemikalien und Feinchemikalien, Synthesebausteine von Wirkstoffen.

Die nachfolgenden Beispiele dienen der näheren Erläuterung der Erfindung, sind jedoch nicht einschränkend auszulegen.

### Ausführungsbeispiel 1:

### Klonierung des Gen für Serratia marcescens Nuklease in einen Bacillus Expressionsvektor mit einem Bacillus subtilis Promotor

Mit zwei Primern (SEQ-ID 173 und SEQ-ID 174) wird das Gen der *Serratia marcescens* Nuklease inklusive des Signalpeptides und einer *Bacillus subtilis* Ribosomenbindestelle (SEQ-ID 175) aus dem an die Codon-Usage von *Bacillus subtilis* angepassten synthetisch erstellten Gen durch eine PCR unter den nachfolgenden Bedingungen amplifiziert und die Sequenz (SEQ-ID 176) erhalten. Auf der amplifizierten Sequenz sind ebenfalls die Schnittstellen für Pael stromaufwärts des Nukleasegens und Pstl stromabwärts des Nukleasegens vorhanden.

### 1.1 PCR:

| | | | |
|---|---|---|---|
| PCR-Ansatz: | 20 µl | 5 x Phusion Polymerase Puffer (Finnzymes) | |
| | 2 µl | dNTPs (je 10 mmol/Liter) | |
| | 100 pmol | Primer 1 | (SEQ-ID 173) |
| | 100 pmol | Primer 2 | (SEQ-ID 174) |
| | 1 µl | Ursprungssequenz (20 ng) | |
| | 1 U | Phusion Polymerase (Finnzymes) | |
| | ad 100 µl | H₂O dest. | |

| | | | |
|---|---|---|---|
| Temperaturprofil der PCR: | 1 min / 98 °C | | |
| | 1. | 10 sec / 98 °C (Denaturierung) | |
| | 2. | 20 sec / 52 °C (Anlagerung) | 30 x |
| | 3. | 2 min 20 sec / 72 °C (Elongation) | |
| | 7 min / 72 °C | | |

Die resultierenden PCR-Produkte werden mittel des High Pure PCR Product Purification Kit (Roche, Diagnostics GmbH; Mannheim) nach Herstellervorschrift gereinigt.

### 1.2 Restriktionsverdau:

Als Expressionsvektor kann jeder in *Bacillus species* replizierender Vektor gewählt werden. Zur Vorbereitung wird in diesen Vektor ein Promotor (SEQ-ID 171) mit nachfolgender multipler Klonierungsstelle (SEQ-ID 177) eingefügt und ggf. bereits vorhandene und störende Promotoren und Restriktionsschnittstellen entfernt. Methoden zur Erzeugung eines solchen leeren Expressionsvektors sind dem Fachmann bekannt und gehören zur Standard-Molekularbiologie.

Zur Klonierung des Gens in einen entsprechend vorbereiteten Expressionsvektor, werden das PCR-Produkt und der Vektor mit den Restriktionsendonukleasen Pael und Pstl (alle MBI Fermentas, Vilnius, Litauen) wie folgt inkubiert:

**Restriktionsverdau-Ansätze:**

| PCR-Produkte: | | Vektor: | |
|---|---|---|---|
| | | | |
| 2,4 µg | PCR-Produkt | 8 µg | Vektor |
| 5 µl | 10x Tango (MBI) | 4 µl | 10x Tango (MBI) |
| 30 U | Pael | 20 U | Pael |
| 10 U | Pstl | 10 U | Pstl |
| ad 50 µl | H₂O dest. | ad 40 µl | H₂O dest. |

Die Restriktionsverdau-Ansätze werden 2 h bei 37 °C inkubiert. Zu dem "Vektor-Ansatz" wird anschließend zur Dephosphorylierung 1 U SAP (MBI Fermentas, Vilnius, Litauen) hinzu gegeben und für weitere 30 min bei 37 °C inkubiert. Danach wird erneut 1 U SAP (MBI Fermentas, Vilnius, Litauen) hinzu gegeben und nochmals 30 min bei 37 °C inkubiert Anschließend werden die Enzyme für 20 min bei 80 °C inaktiviert, mit Phenol und Chloroform extrahiert und der Ansatz zum Ankonzentrieren mit PEG gefällt. Das geschnittene PCR_Produkt wird mittels des Promega Wizard SV Gel and PCR Clean-Up System (Promega GmbH, Mannheim) aufgereinigt.

### 1.3 Ligation, Transformation von B. subtilis und Plasmid-Reisolation

Die Vektor-DNA und das PCR-Produkt werden durch die Inkubation mit T4-DNA-Ligase wie folgt miteinander verbunden:

| | | |
|---|---|---|
| Ligase-Ansatz: | 50 fmol | Vektor-DNA |
| | 150 fmol | PCR-Produkt |
| | 2 µl | 10x Ligase-Puffer (MBI) |
| | 1 µl | T4-DNA-Ligase |
| | ad 20 µl | H₂O dest. |

Die Ansätze werden 16 h bei 16 °C inkubiert und anschließend wurde das Enzym durch 10-minütige Inkubation bei 65 °C inaktiviert. Die Ansätze werden mit Phenol und Chloroform extrahiert, mit Ethanol gefällt und in 20µl deionisierten Wasser aufgenommen. Vor dem Transformieren werden 20µl 2xSMM zu den Ansätzen gegeben. Dann werden die Ansätze verwendet, um *Bacillus subtilis* nach der PEG-Protoplasten-Methode (Chang and Cohen, 1979) zu transformieren. Für die Isolierung der Plasmide wird das High Pure Plasmid Isolation Kit (Roche, Diagnostics GmbH; Mannheim) nach Angaben des Herstellers verwendet. Die so isolierten Plasmide werden durch Sequenzierung des klonierten Gens bezüglich ihrer korrekten Konstruktion getestet.

### Ausführungsbeispiel 2:

### Klonierung des Gens für Serratia marcescens Nuklease in einen Bacillus Expressionsvektor mit einem Bacillus amyloliquefaciens Promotor

Mit zwei Primern (SEQ-ID 178 und SEQ-ID 179) wird das an die Codon-Usage von *Bacillus subtilis* angepasste Gen der *Serratia marcescens* Nuklease inklusive des Signalpeptides und einer *Bacillus subtilis* Ribosomenbindestelle durch eine PCR und dem unter 1. konstruierten Plasmid als Template unter den nachfolgenden Bedingungen amplifiziert. Gleichzeitig sind auf diesem PCR-Produkt (SEQ-ID 180) Schnittstellen für Pael und Bpu1102l enthalten.

### 2.1 PCR:

| | | | |
|---|---|---|---|
| PCR-Ansatz: | 20 µl | 5 x Phusion Polymerase Puffer (Finnzymes) | |
| | 2 µl | dNTPs (je 10 mmol/Liter) | |
| | 200 pmol | Primer 1 | (SEQ-ID 178) |
| | 200 pmol | Primer 2 | (SEQ-ID 179) |
| | 1 µl | Templatevektor aus Beispiel 1 (20 ng) | |
| | 1 U | Phusion Polymerase (Finnzymes) | |
| | ad 100 µl | H₂O dest. | |

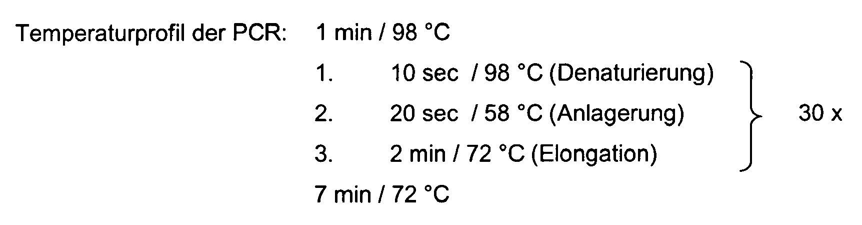

Die resultierenden PCR-Produkte werden mittel des High Pure PCR Product Purification Kit (Roche, Diagnostics GmbH; Mannheim) nach Herstellervorschrift gereinigt.

### 2.2 Restriktionsverdau:

Als Expressionsvektor kann jeder in *Bacillus species* replizierender Vektor gewählt werden. Zur Vorbereitung wird in diesen Vektor ein Promotor (SEQ-ID 172) mit nachfolgender multipler Klonierungsstelle (SEQ-ID 181) eingefügt und ggf. bereits vorhandene und störende Promotoren und Restriktionsschnittstellen entfernt. Methoden zur Erzeugung eines solchen leeren Expressionsvektors sind dem Fachmann bekannt und gehören zur Standard-Molekularbiologie.

Zur Klonierung des Genes in einen entsprechend vorbereiteten Expressionsvektor, werden das PCR-Produkt und der Vektor mit den Restriktionsendonukleasen Pael und Bpu1102l (alle MBI Fermentas, Vilnius, Litauen) wie folgt inkubiert:

**Restriktionsverdau-Ansätze:**

| PCR-Produkte: | | Vektor: | |
|---|---|---|---|
| | | | |
| 1,5 µg | PCR-Produkt | 4 µg | Vektor |
| 5,5 µl | 10x Tango (MBI) | 5 µl | 10x Tango (MBI) |
| 20 U | Pael | 20 U | Pael |
| 10 U | Bpu1102l | 10 U | Bpu1102l |
| ad 55 µl | H₂O dest. | ad 50 µl | H₂O dest. |

Die Restriktionsverdau-Ansätze werden 2 h bei 37 °C inkubiert. Zu dem "Vektor-Ansatz" wird anschließend zur Dephosphorylierung 1 U CIAP (MBI Fermentas, Vilnius, Litauen) und 5µl CIAP Puffer 10x (MBI Fermentas, Vilnius, Litauen) hinzu gegeben und für weitere 30 min bei 37 °C inkubiert. Danach wird erneut 1 U CIAP (MBI Fermentas, Vilnius, Litauen) hinzu gegeben und nochmals 30 min bei 37 °C inkubiert Anschließend werden 5mM EDTA pH 8 hinzugegeben und die Enzyme für 30 min bei 80 °C inaktiviert. Das geschnittene PCR_Produkt wird mittels des Promega Wizard SV Gel and PCR Clean-Up System (Promega GmbH, Mannheim) aufgereinigt.

### 2.3 Ligation, Transformation von B. subtilis und Plasmid-Reisolation

Die Vektor-DNA und das PCR-Produkt werden durch die Inkubation mit T4-DNA-Ligase wie folgt miteinander verbunden:

| | | |
|---|---|---|
| Ligase-Ansatz: | 50 fmol | Vektor-DNA |
| | 150 fmol | PCR-Produkt |
| | 6 µl | 10x Ligase-Puffer (MBI) |
| | 2 µl | T4-DNA-Ligase |
| | ad 60 µl | H₂O dest. |

Die Ansätze werden 16 h bei 16 °C inkubiert und anschließend wurde das Enzym durch 10-minütige Inkubation bei 65 °C inaktiviert. Die Ansätze werden mit Phenol und Chloroform extrahiert, mit Ethanol gefällt und in 20µl deionisierten Wasser aufgenommen. Vor dem Transformieren werden 20µl 2xSMM zu den Ansätzen gegeben. Dann werden die Ansätze verwendet um *Bacillus subtilis* nach der PEG-Protoplasten-Methode (Chang and Cohen,1979) zu transformieren. Für die Isolierung der Plasmide wird das High Pure Plasmid Isolation Kit (Roche, Diagnostics GmbH; Mannheim) nach Angaben des Herstellers verwendet.

### Ausführungsbeispiel 3:

### Klonierung zur Fusion der Nuklease aus Serratia marcescens mit einer AmyE Sekretionssequenz

Mit zwei Primern (SEQ-ID 174 und SEQ-ID 182) wird das an die Codon-Usage von *Bacillus subtilis* angepasste Gen der *Serratia marcescens* Nuklease exklusive des Signalpeptides durch eine PCR und SEQ-ID 176 als Template-Sequenz unter den nachfolgenden Bedingungen amplifiziert.

### 3.1 PCR:

### 3.1.1. Nuklease PCR

| | | |
|---|---|---|
| PCR-Ansatz: | 20 µl | 5 x Phusion Polymerase Puffer (Finnzymes) |
| | 2 µl | dNTPs (je 10 mmol/Liter) |
| | 50 pmol | Primer 1 (SEQ-ID 174) |
| | 50 pmol | Primer 2 (SEQ-ID 182) |
| | 2 µl | Template-Sequenz (20 ng) |
| | 1 U | Phusion Polymerase (Finnzymes) |
| | ad 100 µl | H₂O dest. |

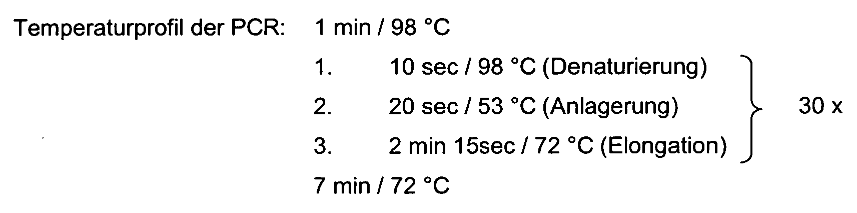

Mit zwei Primern (SEQ-ID 178 und SEQ-ID 183) wird die für die Sekretionssequenz von AmyE aus *Bacillus subtilis* kodierende Sequenz durch eine PCR und die synthetisch erstellte Sequenz SEQ-ID 184 als Template-Sequenz unter den nachfolgenden Bedingungen amplifiziert.

### 3.1.2. AmyE Signalsequenz PCR

| | | |
|---|---|---|
| PCR-Ansatz: | 20 µl | 5 x Phusion Polymerase Puffer (Finnzymes) |
| | 2 µl | dNTPs (je 10 mmol/Liter) |
| | 50 pmol | Primer 1 (SEQ-ID 178) |
| | 50 pmol | Primer 2 (SEQ-ID 183) |
| | 2 µl | Template-Sequenz (20 ng) |
| | 1 U | Phusion Polymerase (Finnzymes) |
| | ad 100 µl | H₂O dest. |

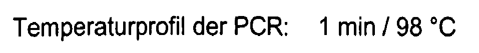

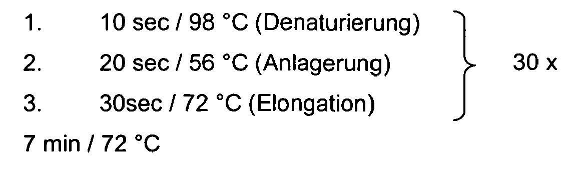

Die resultierenden PCR-Produkte werden mittel des High Pure PCR Product Purification Kit (Roche, Diagnostics GmbH; Mannheim) nach Herstellervorschrift gereinigt.

### 3.1.3. Fusions-PCR AmyE Sekretionssequenz mit Nuklease aus Serratia marcescens

| | | |
|---|---|---|
| PCR-Ansatz: | 20 µl | 5 x Phusion Polymerase Puffer (Finnzymes) |
| | 2 µl | dNTPs (je 10 mmol/Liter) |
| | 5,5 µl | PCR Produkt 3.1.1 (600 fmol) |
| | 1 µl | PCR Produkt 3.1.2 (600 fmol) |
| | 1 U | Phusion Polymerase (Finnzymes) |
| | ad 98 µl | H₂O dest. |

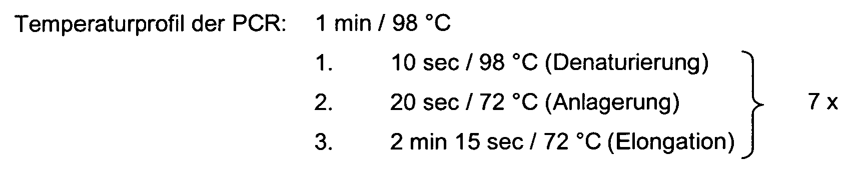

Direkt nach Ablauf der sieben Zyklen wurden je 1µl (100 pmol) Primer 1 (SEQ-ID 178) und Primer 2 (SEQ-ID 174) zum PCR-Ansatz gegeben und eine weitere PCR mit folgendem Temperaturprofil durchgeführt:

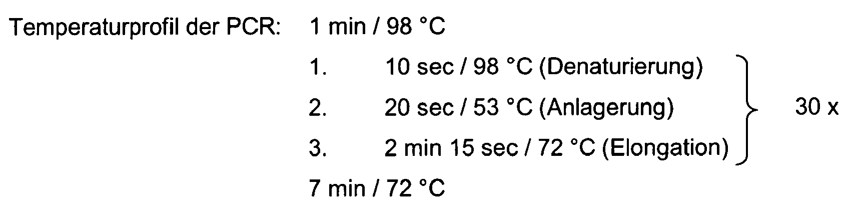

Die resultierenden PCR-Produkte werden mittel des High Pure PCR Product Purification Kit (Roche, Diagnostics GmbH; Mannheim) nach Herstellervorschrift gereinigt und wie unter 1.2 und 1.3 beschrieben, in einen Expressionsvektor, der in *Bacillus species* repliziert und einen Promotor mit nachfolgender multiplen Klonierungsstelle (SEQ-ID 177) trägt, kloniert.

### Ausführungsbeispiel 4:

### Klonierung zur Fusion der Nuklease aus Serratia marcescens mit einer AmyE Sekretionssequenz und einem N-terminalem Affinitäts-Tag (His-Tag) am sekretierten Protein

Mit zwei Primern (SEQ-ID 185 und SEQ-ID 179) wird das an die Codon-Usage von *Bacillus subtilis* angepasste Gen der *Serratia marcescens* Nuklease exklusive des Signalpeptides durch eine PCR und dem unter 3. konstruierten Plasmid als Template unter den nachfolgenden Bedingungen amptifiziert.

### 4.1 PCR:

### 4.1.1. His-Nuklease PCR

| | | |
|---|---|---|
| PCR-Ansatz: | 20 µl | 5 x Phusion Polymerase Puffer (Finnzymes) |
| | 2 µl | dNTPs (je 10 mmol/Liter) |
| | 50 pmol | Primer 1 (SEQ-ID 185) |
| | 50 pmol | Primer 2 (SEQ-ID 179) |
| | 2 µl | Template-Sequenz (20 ng) |
| | 1 U | Phusion Polymerase (Finnzymes) |
| | ad 100 µl | H₂O dest. |

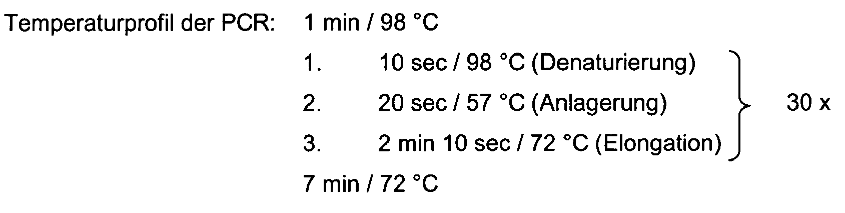

Die resultierenden PCR-Produkte werden mittel des High Pure PCR Product Purification Kit (Roche, Diagnostics GmbH; Mannheim) nach Herstellervorschrift gereinigt.

### 4.1.2. Fusions-PCR AmyE Signalseguenz mit His-Tag und Nuklease aus Serratia marcescens

| | | |
|---|---|---|
| PCR-Ansatz: | 20 µl | 5 x Phusion Polymerase Puffer (Finnzymes) |
| | 2 µl | dNTPs (je 10 mmol/Liter) |
| | 1,5 µl | PCR Produkt 4.1.1 (600 fmol) |

| | | |
|---|---|---|
| | 1 µl | PCR Produkt 3.1.2 (600 fmol) |
| | 1 U | Phusion Polymerase (Finnzymes) |
| | ad 98 µl | H₂O dest. |

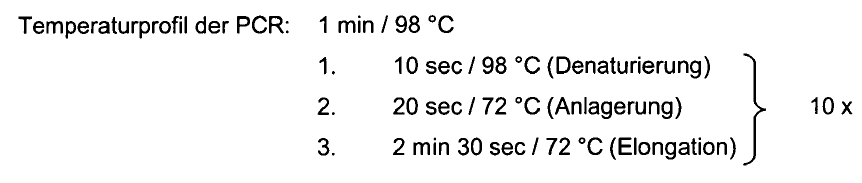

Direkt nach Ablauf der zehn Zyklen wurden je 1µl (100 pmol) Primer 1 (SEQ-ID 178) und Primer 2 (SEQ-ID 179) zum PCR-Ansatz gegeben und eine weitere PCR mit folgendem Temperaturprofil durchgeführt:

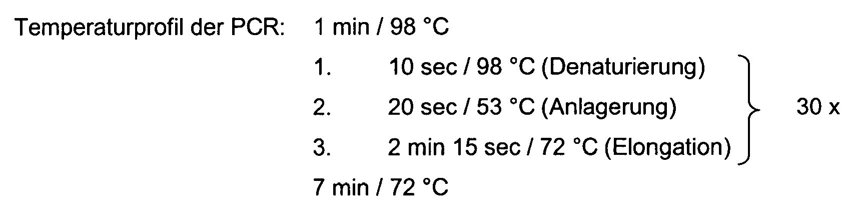

Die resultierenden PCR-Produkte werden mittel des High Pure PCR Product Purification Kit (Roche, Diagnostics GmbH; Mannheim) nach Herstellervorschrift gereinigt und wie unter 1.2 und 1.3 beschrieben, in einen Expressionsvektor, der in *Bacillus species* repliziert und einen Promotor mit nachfolgender multiplen Klonierungsstelle (SEQ-ID 177) trägt, kloniert.

### Ausführungsbeispiel 5:

### Erstellung der Expressionsplasmide für die Sekretion der Nuklease aus Serratia marcescens

Die folgenden Plasmidkonstrukte wurden nach oder entsprechend der Ausführungsbeispiele 1-4 molekularbiologisch konstruiert und durch Sequenzierung verifiziert:
1. Maltose induzierbarer Promotor aus *B. subtilis* + native Nuklease (Codon optimiert), vgl. Ausführungsbeispiel 1
2. Maltose induzierbarer Promotor aus *B*. *amyloliquefaciens* + native Nuklease inkl. eigener Signalsequenz (Codon optimiert), vgl. Ausführungsbeispiel 2
3. Maltose induzierbarer Promotor aus *B. subtilis* + an AmyE Leadersequenz fusionierte Nuklease (Codon optimiert), vgl. Ausführungsbeispiel 3
4. Maltose induzierbarer Promotor aus *B*. *subtilis* + native Nuklease (Codon optimiert) inkl. eigener Signalsequenz mit C-terminalem His-Tag (GGHHHHHHH), analog zu Ausführungsbeispiel 1 und 4
5. Maltose induzierbarer Promotor aus *B. amyloliquefaciens* + an AmyE Leadersequenz fusionierte Nuklease (Codon optimiert), analog zu Ausführungsbeispiel 2 und 3
6. Maltose induzierbarer Promotor aus *B*. *subtilis* + an AmyE Leadersequenz fusionierte Nuklease (Codon optimiert) mit N-terminalem His-Tag (DHHHHHHGG), vgl. Ausführungsbeispiel 4
7. Maltose induzierbarer Promotor aus *B. amyloliquefaciens* + an AmyE Leadersequenz fusionierte Nuklease (Codon optimiert) mit N-terminalem His-Tag (DHHHHHHGG), analog zu Ausführungsbeispiel 2 und 4
8. Wachtumsphasen induzierter *npr* Promotor aus *B. amyloliquefaciens* + an AmyE Leadersequenz fusionierte Nuklease (Codon optimiert), analog zu Ausführungsbeispiel 2 und 3

### Ausführungsbeispiel 6:

### Vergleichende Expressionsuntersuchungen mit den unter 5. erstellten Expressionskonstrukten

Die Plasmidkonstrukte werden in *B. subtilis* wt168 trpC2 *aprE nprE epr amyE bglC* sowie in *B. amyloliquefaciens amy2 npr1* und *amy2 npr1 apr::kan* auf Expression getestet.
a) Dabei werden unterschiedliche Flüssigmedien für die Expression verwendet:
Medium 1:
   LB Medium (10 g/l Trypton, 5 g/l Hefe-Extrakt, 10 g/l NaCl, pH 7,0) mit 5µg/ml Erythromycin +0,1% Glukose;
   Für die Konstrukte mit den Maltose induzierbaren Promotoren wird die Expression durch Zugabe von Maltose (Endkonzentration 1%) beim Übergang zur Stationärphase induziert.
Medium 2:
   5% Maltose +0,1% Glukose + 2% Sojapepton + 2,5% Solulys 095E + 0,5% (NH₄)₂SO₄ + 0,1% KCl + 0,05% Mg₂SO₄·7H₂O
Medium 3:
   10% Maltose + 0,1% Glukose + 2% Sojapepton + 2,5% Solulys 095E + 0,5% (NH₄)₂SO₄ + 0,1% KCl + 0,05% Mg₂SO₄ · 7H₂O
Medium 4:
   100mM Tris-HCl pH7,5 + 10% Maltose + 0,1% Glukose + 2% Sojapepton + 2,5% Solulys 095E + 0,5% (NH₄)₂SO₄ + 0,1% KCl + 0,05% Mg₂SO₄ · 7H₂O

b) Für die Kultivierung von Vorkulturen wird LB Medium mit 2% Glukose und 5µg/ml Erythromycin verwendet.
c) Für die Stammhaltung (Glycerolstock) wird LB Medium mit 2% Glukose und 5µg/ml Erythromycin verwendet. Das Medium wird mit einer einzelnen Kolonie von Platte aus beimpft. Die Kulturen werden in Kulturröhrchen über Nacht bei 30°C unter Schütteln (200rpm) inkubiert. Am nächsten Morgen wird zu 600µl Kultur 200µl 60% steriles Glycerin gegeben. Die Ansätze wurden gemischt und in flüssigem Stickstoff eingefroren. Die Lagerung erfolgte bei -80°C.
d) Für die Propagierung der Stämme auf Platten wird LB Medium mit 5µg/ml Erythromycin + 2% Glukose + 1,5% Agar verwendet.
e) Für die Detektion von Nuklease positiven Kolonien auf Platte wird LB Medium mit 5µg/ml Erythromycin + 0,1% Glukose + 1% Maltose + 0,2% Heringssperma-DNA (AppliChem A2160) + 0,2% RNA aus Hefe (Roche 109223) (für Aktivität) + 1,5% Agar verwendet. Nuklease positive Kolonien werden durch Fluten der Platte mit 1 N HCl nach 2 Tagen Inkubation sichtbar gemacht. Dabei werden positive Kandidaten von einem klaren Hof umgeben.
f) Für Expressionsversuche werden die Kulturen bei 37°C inkubiert. Flüssigkulturen werden dabei mit 150 U/min horizontal geschüttelt. Vorkulturen werden über Nacht bei 30°C inkubiert.
g) Expressionsversuche
i. Medium 1 *Bacillus subtilis* oder *Bacillus amyloliquefaciens* mit Plasmidkonstrukten 1-7
   Ausgehend von einer Vorkultur werden 100ml Medium 1 mit einem ml Vorkultur beimpft. Die Wachstumskurve wird bestimmt und beim Eintreten der Kultur in die Stationärphase, werden dem Medium 2 ml 50% Maltose (EK: 1%) zugegeben. Die Kultur wird über Nacht weiterhin unter Schütteln bei 37°C inkubiert. Am nächsten Morgen wird die Nukleaseaktivität im Überstand bestimmt.
ii. Medium 1 *Bacillus subtilis* oder *Bacillus amyloliquefaciens* mit Plasmidkonstrukt 8
   Ausgehend von einer Vorkultur werden 100ml Medium 1 mit einem ml Vorkultur beimpft. Die Kultur wird für 24 Stunden unter Schütteln bei 37°C inkubiert. Am nächsten Morgen wird die Nukleaseaktivität im Überstand bestimmt.
iii. Medium 2, 3, 4 *Bacillus subtilis* oder *Bacillus amyloliquefaciens* mit Plasmidkonstrukten 1-8
   Ausgehend von einer Vorkultur werden 100ml Medium 1 mit einem ml Vorkultur beimpft. Die Kultur wird über ein Woche weiterhin unter Schütteln bei 37°C inkubiert. Jeden Tag wird die Nukleaseaktivität im Überstand bestimmt.

h) Nukleaseaktivitätsmessungen
Chemikalien
- DNA (salmon testes) - Sigma D1626 -> für Assay
- RNA (Hefe) Roche 109223 -> für Platten
- DNA (Salmon Sperm) AppliChem A2160-> für Platten
- BSA (10 mg/ml) NEB B9001S

Puffer, Lösungen
• 1 M Tris-HCl pH 8,2; autoklavieren
• 100 mM MgCl₂; autoklavieren
• Assay-Puffer (immer frisch herstellen)

| Beispiel für 100 ml | | |
|---|---|---|
| | Endkonzentration | Stammlösung |
| Tris-HCl | 50 mM | 5 ml |
| BSA | 0,1 mg/ml | 1 ml |
| MgCl₂ | 1 mM | 1 ml |
| mit sterilem dest. Wasser auf 100 ml auffüllen | | |

• Substratpuffer (1 mg/ml DNA Sigma D1626 in Assay-Puffer).
• 4% Perchlorsäure (10 ml 70%-ige Perchlorsäure langsam in 165 ml Wasser geben)
100 µl Substratpuffer werden mit 20 µl DNase-haltiger Lösung versetzt. Ist eine Verdünnung der DNase notwendig, so wird das Enzym in Assay-Puffer verdünnt. Je nach Aktivität und Reinheit wird unterschiedlich stark verdünnt werden müssen. Als Leerwert wird die *S. marescens* Nuklease durch Assay-Puffer ersetzt.
Die Lösung wird genau 20 Minuten bei 37°C inkubiert und die Reaktion anschließend durch Zugabe von 100 µl 4% Perchlorsäure abgestoppt. Die Lösung wird sofort anschließend 10 Minuten auf Eis inkubiert, um eine vollständige Ausfällung der nicht umgesetzten DNA zu gewährleisten. Anschließend wird die ausgefällte DNA abzentrifugiert (16.000 x g; 10 min; 4°C). Vom Überstand werden 150 µl abgenommen und 1:5 verdünnt gegen Wasser gemessen. Dazu werden die 150 µl Überstand mit 600 µl Wasser gemischt und die Extinktion in einer Quarzküvette Photometer bei 260 nm gemessen, welches vorher gegen Wasser abgeglichen worden ist.
Ein Unit entspricht dabei der Menge an Enzym, die eine Absorptionsänderung bei 260nm von 1 in 60 Minuten bewirkt.
i) Erhaltene *S. marescens* Nuklease-Expressionsraten (MU pro Liter Überstand):

| Medium | 1 | 2 | 3 | 4 |
|---|---|---|---|---|
| *B. subtilis aprE nprE epr amyE bglc* mit Plasmidkonstrukt 1 | 0,4 | | | |
| *B. subtilis aprE nprE epr amyE bglC* mit Plasmidkonstrukt 2 | 0,6 | | | |
| *B. subtilis aprE nprE epr amyE bglC* mit Plasmidkonstrukt 3 | 2,5 | | | |
| *B. subtilis aprE nprE epr amyE bglC* mit Plasmidkonstrukt 4 | 0,2 | | | |
| *B. subtilis aprE nprE epr amyE bglC* mit Plasmidkonstrukt 5 | 3,7 | 0,7 | | |
| *B. subtilis aprE nprE epr amyE bglC* mit Plasmidkonstrukt 6 | 1,6 | | | |
| *B. subtilis aprE nprE epr amyE bglC* mit Plasmidkonstrukt 8 | 1,0 | 0,3 | | |
| *B. amyloliguefaciens amy2 npr1* mit Plasmidkonstrukt 1 | 0,9 | | | |
| *B. amyloliquefaciens amy2 npr1* mit Plasmidkonstrukt 2 | 1,1 | | | |
| *B. amyloliquefaciens amy2 npr1* mit Plasmidkonstrukt 3 | 0,5 | | | |
| *B. amyloliquefaciens amy2 npr1* mit Plasmidkonstrukt 5 | 1,3 | 25,7 | 15,1 | 26,4 |
| *B. amyloliquefaciens amy2 npr1* mit Plasmidkonstrukt 8 | 1,0 | 12,1 | | |
| *B. amyloliquefaciens amy2 npr1 apr::kan* mit Plasmidkonstrukt 5 | | | 21,1 | 27,7 |
| *B. amyloliquefaciens amy2 npr1 apr::kan* mit Plasmidkonstrukt 6 | | | | 10,6 |
| *B. amyloliquefaciens amy2 npr1 apr::kan* mit Plasmidkonstrukt 7 | | | | 11,9 |

### Ausführungsbeispiel 7:

*Heterologe Expression der Serratia marcescens Nuklease in Escherichia coli.*

### Klonierung und Expression

Es wurden Expressionsvektoren konstruiert durch Insertion eines offenen DNA Fragments in das Plasmid pBR327 (DSMZ, Braunschweig). Das DNA Fragment umfasste den offenen Leserahmen der *Serratia marcescens* Nuklease inklusive derer nativer Signalsequenz unter der Kontrolle des nativen Serratia marcescens Nuklease Promotors. Es wurden zwei Varianten konstruiert, die sich in der Orientierung des DNA-Fragments im Bezug auf das Resistenzgen von pBR327 unterscheiden. Die Expression der Nuklease erfolgte in dem *E*. *coli* Stamm MC1000 von (CGSC, New Haven USA) im Schüttelkolben in LB-Medium mit 0,2% Glukose und 100µg/ml Ampicillin bei 37°C für 72h. Als Kontrolle wurde der Stamm MC1000 mit dem Leerplasmid pBR327 verwendet. Zu unterschiedlichen Zeitpunkten wurden Proben der Kulturen entnommen. Die Zellen wurden durch Zentrifugation abgetrennt und die Nuklease Aktivität im Überstand bestimmt.

Die Bestimmung der exprimierten Enzym-Aktivitäten erfolgen wie unter h) in Ausführungsbeispiel 6 beschrieben.

Die folgenden maximalen Expressionsausbeuten wurden erreicht:

| | MU pro Liter Kultur Überstand |
|---|---|
| MC1000 pBR327 | 0,042 |
| MC1000 pBR327 + Nuklease Variante 1 | 0,066 |
| MC1000 pBR327 + Nuklease Variante 2 | 0,074 |

In einem zweiten Ansatz wurde die Serratia marcescens Nuklease im Fermenter exprimiert. Als Medium wurde LB-Medium mit 0,2% Glukose und 100µg/ml Ampicillin verwendet. Die Wachstumstemperatur betrug 35°C. Der pH Wert wurde auf 8,4 eingestellt. Die Belüftung erfolgte bei 500 rpm mit 1vvm Luft. Die Fermentation erfolgte für 72h. Zu unterschiedlichen Zeitpunkten wurden Proben der Kulturen entnommen. Die Zellen wurden durch Zentrifugation abgetrennt und die Nuklease Aktivität im Überstand bestimmt.

Ebenso wurde der Gehalt an Nuklease Aktivität im Periplasma der Stämme untersucht. Dazu wurde das abgetrennte Pellet der Zellen 0,03M Tris-HCl pH8 20% Saccharose aufgenommen. Für 1g Pellet wurden 80 ml Puffer verwendet. Anschließend wurde EDTA (Endkonzentration 1 mM) zu den Suspensionen gegeben. Die Suspensionen wurde für 10 Minuten unter schütteln inkubiert. Anschließend wurden die Zellen durch Zentrifugation pelletiert. Die Pellets wurden in einem Probenvolumen 4°C kaltem deionisiertem Wasser aufgenommen und bei 4°C für 10 Minuten inkubiert. Die Suspension wurde zentrifugiert und die Nuklease Aktivität im Überstand vermessen.

Die Bestimmung der exprimierten Enzym-Aktivitäten erfolgen wie unter h) in Ausführungsbeispiel 6 beschrieben.

Die folgenden maximalen Expressionsausbeuten wurden erreicht:

| | MU pro Liter Kultur Überstand | MU pro Liter Kultur Periplasma |
|---|---|---|
| MC1000 pBR327 | 0,032 | 0,002 |
| MC1000 pBR327 + Nuklease Variante 1 | 0,062 | 0,023 |
| MC1000 pBR327 + Nuklease Variante 2 | 0,086 | 0,025 |

## Patentansprüche

1. Ein Verfahren zur Herstellung einer Nuklease eines gram-negativen Bakteriums oder einer Nuklease-Präparation enthaltend eine Nuklease eines gram-negativen Bakteriums umfassend die Expression der Nuklease in einem gram-positiven Bakterium der Gattung *Bacillus* und die anschließende Sekretion der Nuklease; wobei
- die Aminosäuresequenz der Nuklease 60% Homologie mit SEQ ID NO:3 aufweist;
- die Expression mit Hilfe eines induzierbaren Promotors gesteuert wird; der
- durch ein äußeres Signal induziert wird; und/oder
- der Maltose-Promotor einer *Bacillus sp.* ist; und
- ein DNA-Segment, enthaltend eine DNA-Sequenz, welche für die Nuklease codiert, und eine DNA-Sequenz, welche für eine Sekretionssequenz codiert, in das gram-positive Bakterium der Gattung *Bacillus* eingebracht werden; wobei die Sekretionssequenz ausgewählt ist aus den Sekretionssequenzen für
- *amyE* aus *B*. *subtilis* gemäß SEQ ID NO:5 oder
- amyE aus *B. amyloliquefaciens* gemäß SEQ ID NO:170.

2. Das Verfahren nach einem der vorstehenden Ansprüche, **dadurch gekennzeichnet, dass** das gram-positive Bakterium der Gattung *Bacillus* ein Protease-armer Wirt ist.

3. Das Verfahren nach einem der vorstehenden Ansprüche, **dadurch gekennzeichnet, dass** die Nuklease zusätzlich einen Affinitäts-Tag aufweist.

4. Das Verfahren nach einem der vorstehenden Ansprüche, **dadurch gekennzeichnet, dass** die Expression mit Hilfe eines Expressionsvektors erfolgt.

5. Das Verfahren nach einem der vorstehenden Ansprüche, **dadurch gekennzeichnet, dass** die Nuklease sowohl DNAse- als auch RNAse-Aktivität aufweist.

6. Das Verfahren nach einem der vorstehenden Ansprüche, **dadurch gekennzeichnet, dass** es keinen Reinigungsschritt umfasst, welcher der gezielten Abtrennung von Endotoxinen dient.

## Claims

1. Method for producing a nuclease of a gram-negative bacterium or a nuclease preparation containing a nuclease of a gram-negative bacterium, comprising expression of said nuclease in a gram-positive bacterium of the genus *Bacillus* and subsequent secretion of said nuclease; wherein
- the amino acid sequence of the nuclease is 60% homologous to SEQ ID NO:3;
- expression is controlled with the aid of an inducible promoter; which
- is induced by an external signal; and/or
- is the maltose promoter of a *Bacillus sp.;* and
- a DNA segment containing a DNA sequence encoding the nuclease and a DNA sequence encoding a secretion sequence are introduced to said gram-positive bacterium of the genus *Bacillus;* wherein said secretion sequence is selected from the secretion sequences for
- *B. subtilis amyE* set forth in SEQ ID NO:5, or
- *B. amyloliquefaciens* amyE set forth in SEQ ID NO:170.

2. Method according to any preceding claim, **characterized in that** the gram-positive bacterium of the genus *Bacillus* is a protease-deficient host.

3. Method according to any preceding claim, **characterized in that** the nuclease additionally has an affinity tag.

4. Method according to any preceding claim, **characterized in that** expression is performed with the aid of an expression vector.

5. Method according to any preceding claim, **characterized in that** the nuclease has both DNase activity and RNase activity.

6. Method according to any preceding claim, **characterized in that** it does not include any purification step for specifically removing endotoxins.

## Revendications

1. Procédé de fabrication d'une nucléase d'une bactérie à Gram négatif ou d'une préparation de nucléase contenant une nucléase d'une bactérie à Gram négatif, comprenant l'expression de la nucléase dans une bactérie à Gram positif du genre *Bacillus,* puis la sécrétion de la nucléase ;
- la séquence d'acides aminés de la nucléase présentant 60 % d'homologie avec SEQ ID NO : 3 ;
- l'expression étant régulée à l'aide d'un promoteur inductible ; qui
- est induit par un signal extérieur ; et/ou
- est le promoteur sensible au maltose d'une *Bacillus sp. ;* et
- un segment d'ADN, contenant une séquence d'ADN qui code pour la nucléase et une séquence d'ADN qui code pour une séquence de sécrétion, étant introduit dans la bactérie à Gram positif du genre *Bacillus ;* la séquence de sécrétion étant choisie parmi les séquences de sécrétion pour
- amyE de *B. subtilis* selon SEQ ID NO : 5 ou
- amyE de *B. amyloliquefaciens* selon SEQ ID NO : 170.

2. Procédé selon l'une quelconque des revendications précédentes, **caractérisé en ce que** la bactérie à Gram positif du genre *Bacillus* est un hôte pauvre en protéases.

3. Procédé selon l'une quelconque des revendications précédentes, **caractérisé en ce que** la nucléase comprend en outre un marqueur d'affinité.

4. Procédé selon l'une quelconque des revendications précédentes, **caractérisé en ce que** l'expression a lieu à l'aide d'un vecteur d'expression.

5. Procédé selon l'une quelconque des revendications précédentes, **caractérisé en ce que** la nucléase présente aussi bien une activité ADNase qu'ARNase.

6. Procédé selon l'une quelconque des revendications précédentes, **caractérisé en ce qu'**il ne comprend pas d'étape de purification servant à la séparation ciblée d'endotoxines.


### SEQUENCE LISTING

<110> c-Lecta
   c-Lecta GmbH
<120> Verfahren zur Herstellung von Nukleasen eines gram-negativen Bakteriums unter Nutzung eines gram-positiven Expressionswirtes
<130> IVOOO6-WO
<150> EP09009992.0
   <151> 2009-08-03
<160> 185
<170> PatentIn version 3.3
<210> 1
   <211> 801
   <212> DNA
   <213> Serratia marcescens
<400> 1
   atgcgcttta acaacaagat gttggccttg gccgccctgc tgttcgccgc gcaggcgtcg      60
   gccgacacgc tcgaatccat cgacaactgc gcggtcggct gcccgaccgg cggcagcagc     120
   aacgtgtcta tcgtgcgcca tgcttatacg ttgaacaaca acagcaccac caagttcgcc     180
   aactgggtgg cctatcacat caccaaagac acgccggcca gcggcaagac gcgcaactgg     240
   aaaaccgatc cggctctcaa tccggcggac actctggcgc ccgccgatta caccggtgcc     300
   aacgccgcgc tgaaggtcga tcgcggtcat caggcgccgc tggcctcgct ggcgggcgtt     360
   tccgactggg aatcgttgaa ctacctgtcc aacatcacgc cgcaaaagtc cgatctgaac     420
   cagggcgcct gggctcggct ggaagatcag gaacgcaagc tgatcgatcg cgccgatatc     480
   tcctcggtct ataccgtgac cgggccgctg tatgagcgcg atatgggcaa actgccgggc     540
   acccagaaag cgcacaccat ccccagcgcc tactggaagg taattttcat caacaacagc     600
   ccggcggtga accactatgc cgccttcctg ttcgaccaga acacgccgaa gggcgccgat     660
   ttctgccaat tccgcgtgac ggtggacgag atcgagaaac gcaccggcct gatcatctgg     720
   gccggtctgc cggacgacgt gcaggcttcg ctgaagagca aaccgggcgt tctgccggag     780
   ttgatgggct gcaaaaactg a                                               801
<210> 2
   <211> 21
   <212> PRT
   <213> Serratia marcescens
<400> 2
   Met Arg Phe Asn Asn Lys Met Leu Ala Leu Ala Ala Leu Leu Phe Ala
   1               5                   10                  15
   Ala Gln Ala Ser Ala
               20
<210> 3
   <211> 245
   <212> PRT
   <213> Serratia marcescens
<400> 3
   Asp Thr Leu Glu Ser Ile Asp Asn Cys Ala Val Gly Cys Pro Thr Gly
   1               5                   10                  15
   Gly Ser Ser Asn Val Ser Ile Val Arg His Ala Tyr Thr Leu Asn Asn
               20                  25                  30
   Asn Ser Thr Thr Lys Phe Ala Asn Trp Val Ala Tyr His Ile Thr Lys
           35                  40                  45
   Asp Thr Pro Ala Ser Gly Lys Thr Arg Asn Trp Lys Thr Asp Pro Ala
       50                  55                  60
   Leu Asn Pro Ala Asp Thr Leu Ala Pro Ala Asp Tyr Thr Gly Ala Asn
   65                  70                  75                      80
   Ala Ala Leu Lys Val Asp Arg Gly His Gln Ala Pro Leu Ala Ser Leu
                   85                  90                  95
   Ala Gly Val Ser Asp Trp Glu Ser Leu Asn Tyr Leu Ser Asn Ile Thr
               100                 105                 110
   Pro Gln Lys Ser Asp Leu Asn Gln Gly Ala Trp Ala Arg Leu Glu Asp
           115                 120                 125
   Gln Glu Arg Lys Leu Ile Asp Arg Ala Asp Ile Ser Ser Val Tyr Thr
       130                 135                 140
   Val Thr Gly Pro Leu Tyr Glu Arg Asp Met Gly Lys Leu Pro Gly Thr
   145                 150                 155                 160
   Gln Lys Ala His Thr Ile Pro Ser Ala Tyr Trp Lys Val Ile Phe Ile
                   165                 170                 175
   Asn Asn Ser Pro Ala Val Asn His Tyr Ala Ala Phe Leu Phe Asp Gln
               180                 185                 190
   Asn Thr Pro Lys Gly Ala Asp Phe Cys Gln Phe Arg Val Thr Val Asp
           195                 200                 205
   Glu Ile Glu Lys Arg Thr Gly Leu Ile Ile Trp Ala Gly Leu Pro Asp
       210                 215                 220
   Asp Val Gln Ala Ser Leu Lys Ser Lys Pro Gly Val Leu Pro Glu Leu
   225                 230                 235                 240
   Met Gly Cys Lys Asn
                   245
<210> 4
   <211> 32
   <212> PRT
   <213> Bacillus subtilis
<400> 4
   Met Lys Lys Lys Lys Thr Trp Lys Arg Phe Leu His Phe Ser Ser Ala
   1               5                   10                  15
   Ala Leu Ala Ala Gly Leu Ile Phe Thr Ser Ala Ala Pro Ala Glu Ala
               20                  25                  30
<210> 5
   <211> 33
   <212> PRT
   <213> Bacillus subtilis
<400> 5
   Met Phe Ala Lys Arg Phe Lys Thr Ser Leu Leu Pro Leu Phe Ala Gly
   1               5                   10                  15
   Phe Leu Leu Leu Phe His Leu Val Leu Ala Gly Pro Ala Ala Ala Ser
               20                  25                  30
   Ala
<210> 6
   <211> 29
   <212> PRT
   <213> Artificial
<220>
   <223> Künstliche Sekretionssequenz
<400> 6
   Met Arg Ser Lys Lys Leu Trp Ile Ser Leu Leu Phe Ala Leu Thr Leu
   1               5                   10                  15
   Ile Phe Thr Met Ala Phe Ser Asn Met Ser Val Gln Ala
               20                  25
<210> 7
   <211> 22
   <212> PRT
   <213> Artificial
<220>
   <223> künstliche Sekretionssequenz
<400> 7
   Met Lys Leu Ala Lys Arg Val Ser Ala Leu Thr Pro Ser Thr Thr Leu
   1               5                   10                  15
   Ala Ile Thr Ala Lys Ala
               20
<210> 8
   <211> 28
   <212> PRT
   <213> Artificial
<220>
   <223> künstliche Sekretionssequenz
<400> 8
   Met Pro Tyr Leu Lys Arg Val Leu Leu Leu Leu Val Thr Gly Leu Phe
   1               5                   10                  15
   Met Ser Leu Phe Ala Val Thr Ala Thr Ala Ser Ala
               20                  25
<210> 9
   <211> 38
   <212> PRT
   <213> Artificial
<220>
   <223> künstliche Sekretionssequenz
<400> 9
   Met Met Arg Arg Arg Lys Arg Ser Asp Met Lys Arg Ser Ile Ser Ile
   1               5                   10                  15
   Phe Ile Thr Cys Leu Leu Ile Thr Leu Leu Thr Met Gly Gly Met Ile
               20                  25                  30
   Ala Ser Pro Ala Ser Ala
           35
<210> 10
   <211> 30
   <212> PRT
   <213> Artificial
<220>
   <223> künstliche Sekretionssequenz
<400> 10
   Met Arg Lys Lys Thr Lys Asn Arg Leu Ile Ser Ser Val Leu Ser Thr
   1               5                   10                  15
   Val Val Ile Ser Ser Leu Leu Phe Pro Gly Ala Ala Gly Ala
               20                  25                  30
<210> 11
   <211> 27
   <212> PRT
   <213> Artificial
<220>
   <223> künstliche Sekretionssequenz
<400> 11
   Met Lys Trp Asn Pro Leu Ile Pro Phe Leu Leu Ile Ala Val Leu Gly
   1               5                   10                  15
   Ile Gly Leu Thr Phe Phe Leu Ser Val Lys Gly
               20                  25
<210> 12
   <211> 32
   <212> PRT
   <213> Artificial
<220>
   <223> künstliche Sekretionssequenz
<400> 12
   Met Gly Asn Thr Arg Lys Lys Val Ser Val Ile Gly Ala Gly Phe Thr
   1               5                   10                  15
   Gly Ala Thr Thr Ala Phe Leu Ile Ala Gln Lys Glu Leu Ala Asp Val
               20                  25                  30
<210> 13
   <211> 37
   <212> PRT
   <213> Artificial
<220>
   <223> künstliche Sekretionssequenz
<400> 13
   Met Lys Asn Arg Leu Phe Ile Leu Ile Cys Phe Cys Val Ile Cys Leu
   1               5                   10                  15
   Phe Leu Ser Phe Gly Gln Pro Phe Phe Pro Ser Met Ile Leu Thr Val
               20                  25                  30
   Gln Ala Ala Lys Ser
           35
<210> 14
   <211> 35
   <212> PRT
   <213> Artificial
<220>
   <223> künstliche Sekretionssequenz
<400> 14
   Met Lys Ile Ser Met Gln Lys Ala Asp Phe Trp Lys Lys Ala Ala Ile
   1               5                   10                  15
   Ser Leu Leu Val Phe Thr Met Phe Phe Thr Leu Met Met Ser Glu Thr
               20                  25                  30
   Val Phe Ala
           35
<210> 15
   <211> 27
   <212> PRT
   <213> Artificial
<220>
   <223> künstliche Sekretionssequenz
<400> 15
   Met Arg Lys Lys Leu Lys Trp Leu Ser Phe Leu Leu Gly Phe Ile Ile
   1               5                   10                  15
   Leu Leu Phe Leu Phe Lys Tyr Gln Phe Ser Asn
               20                  25
<210> 16
   <211> 27
   <212> PRT
   <213> Artificial
<220>
   <223> künstliche Sekretionssequenz
<400> 16
   Met Arg Ile Phe Lys Lys Ala Val Phe Val Thr Met Ile Ser Phe Leu
   1 5 10 15
   Ile Ala Thr Val Asn Val Asn Thr Ala His Ala
   20 25
<210> 17
   <211> 23
   <212> PRT
   <213> Artificial
<220>
   <223> künstliche Sekretionssequenz
<400> 17
   Met Lys Arg Leu Leu Ser Thr Leu Leu Ile Gly Ile Met Leu Leu Thr
   1 5 10 15
   Phe Ala Pro Ser Ala Phe Ala
   20
<210> 18
   <211> 21
   <212> PRT
   <213> Artificial
<220>
   <223> künstliche Sekretionssequenz
<400> 18
   Met Lys Lys Arg Phe Phe Gly Pro Ile Ile Leu Ala Phe Ile Leu Phe
   1 5 10 15
   Ala Gly Ala Ile Ala
   20
<210> 19
   <211> 27
   <212> PRT
   <213> Artificial
<220>
   <223> künstliche Sekretionssequenz
<400> 19
   Met Lys Asn Met Ser Cys Lys Leu Val Val Ser Val Thr Leu Phe Phe
   1 5 10 15
   Ser Phe Leu Thr Ile Gly Pro Leu Ala His Ala
   20 25
<210> 20
   <211> 23
   <212> PRT
   <213> Artificial
<220>
   <223> künstliche Sekretionssequenz
<400> 20
   Met Lys Lys Lys Leu Met Ile Ile Leu Leu Ile Ile Leu Ile Val Ile
   1 5 10 15
   Gly Ala Leu Gly Ala Ala Ala
   20
<210> 21
   <211> 27
   <212> PRT
   <213> Artificial
<220>
   <223> künstliche Sekretionssequenz
<400> 21
   Met Lys Lys Ser Gln Tyr Phe Ile Val Phe Ile Cys Phe Phe Val Leu
   1 5 10 15
   Phe Ser Val His Pro Ile Ala Ala Ala Ala Ala
   20 25
<210> 22
   <211> 26
   <212> PRT
   <213> Artificial
<220>
   <223> künstliche Sekretionssequenz
<400> 22
   Met Arg Lys Asn Arg Ile Leu Ala Leu Phe Val Leu Ser Leu Gly Leu
   1 5 10 15
   Leu Ser Phe Met Val Thr Pro Val Ser Ala
   20 25
<210> 23
   <211> 28
   <212> PRT
   <213> Artificial
<220>
   <223> künstliche Sekretionssequenz
<400> 23
   Met Lys Lys Val Leu Met Ala Phe Ile Ile Cys Leu Ser Leu Ile Leu
   1               5                   10                  15
   Ser Val Leu Ala Ala Pro Pro Ser Gly Ala Lys Ala
               20                  25
<210> 24
   <211> 25
   <212> PRT
   <213> Artificial
<220>
   <223> künstliche Sekretionssequenz
<400> 24
   Met Lys Ser Cys Lys Gln Leu Ile Val Cys Ser Leu Ala Ala Ile Leu
   1               5                   10                  15
   Leu Leu Ile Pro Ser Val Ser Phe Ala
               20                  25
<210> 25
   <211> 24
   <212> PRT
   <213> Artificial
<220>
   <223> künstliche Sekretionssequenz
<400> 25
   Met Arg Ser Tyr Ile Lys Val Leu Thr Met Cys Phe Leu Gly Leu Ile
   1               5                   10                  15
   Leu Phe Val Pro Thr Ala Leu Ala
               20
<210> 26
   <211> 27
   <212> PRT
   <213> Artificial
<220>
   <223> künstliche Sekretionssequenz
<400> 26
   Met Lys Lys Arg Leu Ile Ala Pro Met Leu Leu Ser Ala Ala Ser Leu
   1               5                   10                  15
   Ala Phe Phe Ala Met Ser Gly Ser Ala Gln Ala
               20                  25
<210> 27
   <211> 19
   <212> PRT
   <213> Artificial
<220>
   <223> künstliche Sekretionssequenz
<400> 27
   Met Lys Lys Gln Ile Ile Thr Ala Thr Thr Ala Val Val Leu Gly Ala
   1               5                   10                  15
   Leu Phe Ala
<210> 28
   <211> 26
   <212> PRT
   <213> Artificial
<220>
   <223> künstliche Sekretionssequenz
<400> 28
   Met Lys Lys Lys Leu Ala Ala Gly Leu Thr Ala Ser Ala Ile Val Gly
   1               5                   10                  15
   Thr Thr Leu Val Val Thr Pro Ala Glu Ala
               20                  25
<210> 29
   <211> 38
   <212> PRT
   <213> Artificial
<220>
   <223> künstliche Sekretionssequenz
<400> 29
   Met Arg Asn Glu Arg Arg Lys Lys Lys Thr Leu Leu Leu Thr Ile Leu
   1               5                   10                  15
   Thr Ile Ile Gly Leu Leu Val Leu Gly Thr Gly Gly Tyr Ala Tyr Tyr
               20                  25                  30
   Leu Trp His Lys Ala Ala
           35
<210> 30
   <211> 35
   <212> PRT
   <213> Artificial
<220>
   <223> künstliche Sekretionssequenz
<400> 30
   Met Asp Thr Thr Thr Ala Lys Gln Ala Ser Thr Lys Phe Val Val Leu
   1               5                   10                  15
   Gly Leu Leu Leu Gly Ile Leu Met Ser Ala Met Asp Asn Thr Ile Val
               20                  25                  30
   Ala Thr Ala
           35
<210> 31
   <211> 40
   <212> PRT
   <213> Artificial
<220>
   <223> künstliche Sekretionssequenz
<400> 31
   Met Ala Arg Lys Lys Lys Lys Lys His Glu Asp Glu His Val Asp Glu
   1               5                   10                  15
   Ser Trp Leu Val Pro Tyr Ala Asp Ile Leu Thr Leu Leu Leu Ala Leu
               20                  25                  30
   Phe Ile Val Leu Tyr Ala Ser Ser
           35                  40
<210> 32
   <211> 34
   <212> PRT
   <213> Artificial
<220>
   <223> künstliche Sekretionssequenz
<400> 32
   Met Lys Leu Val Pro Arg Phe Arg Lys Gln Trp Phe Ala Tyr Leu Thr
   1               5                   10                  15
   Val Leu Cys Leu Ala Leu Ala Ala Ala Val Ser Phe Gly Val Pro Ala
               20                  25                  30
   Lys Ala
<210> 33
   <211> 23
   <212> PRT
   <213> Artificial
<220>
   <223> künstliche Sekretionssequenz
<400> 33
   Met Pro Asn Lys Arg Leu Met Leu Leu Leu Leu Cys Ile Ile Ile Leu
   1               5                   10                  15
   Val Ala Met Ile Gly Phe Ser
               20
<210> 34
   <211> 28
   <212> PRT
   <213> Artificial
<220>
   <223> künstliche Sekretionssequenz
<400> 34
   Met Arg Asn Leu Thr Lys Thr Ser Leu Leu Leu Ala Gly Leu Cys Thr
   1               5                   10                  15
   Ala Ala Gln Met Val Phe Val Thr His Ala Ser Ala
               20                  25
<210> 35
   <211> 27
   <212> PRT
   <213> Artificial
<220>
   <223> künstliche Sekretionssequenz
<400> 35
   Met Gly Leu Gly Lys Lys Leu Ser Val Ala Val Ala Ala Ser Phe Met
   1               5                   10                  15
   Ser Leu Ser Ile Ser Leu Pro Gly Val Gln Ala
               20                  25
<210> 36
   <211> 29
   <212> PRT
   <213> Artificial
<220>
   <223> künstliche Sekretionssequenz
<400> 36
   Met Lys Lys Trp Met Ala Gly Leu Phe Leu Ala Ala Ala Val Leu Leu
   1               5                   10                  15
   Cys Leu His Val Pro Gln Gln Ile Gln Gly Ala Ser Ser
               20                  25
<210> 37
   <211> 29
   <212> PRT
   <213> Artificial
<220>
   <223> künstliche Sekretionssequenz
<400> 37
   Met Lys Lys Ser Ile Lys Leu Tyr Val Ala Val Leu Leu Leu Phe Val
   1               5                   10                  15
   Val Ala Ser Val Pro Tyr Met His Gln Ala Ala Leu Ala
               20                  25
<210> 38
   <211> 34
   <212> PRT
   <213> Artificial
<220>
   <223> künstliche Sekretionssequenz
<400> 38
   Met Ile Gln Met Pro Lys Lys Asn Lys Phe Met Asn Arg Gly Ala Ala
   1               5                   10                  15
   Ile Leu Ser Ile Cys Phe Ala Leu Phe Phe Phe Val Ile Leu Gly Arg
               20                  25                  30
   Met Ala
<210> 39
   <211> 26
   <212> PRT
   <213> Artificial
<220>
   <223> künstliche Sekretionssequenz
<400> 39
   Met Thr Met Leu Arg Lys Ile Ile Gly Trp Ile Leu Leu Leu Cys Ile
   1               5                   10                  15
   Ile Pro Leu Phe Ala Phe Thr Val Ile Ala
               20                  25
<210> 40
   <211> 21
   <212> PRT
   <213> Artificial
<220>
   <223> künstliche Sekretionssequenz
<400> 40
   Met Lys Lys Val Met Leu Ala Thr Ala Leu Phe Leu Gly Leu Thr Pro
   1               5                   10                  15
   Ala Gly Ala Asn Ala
               20
<210> 41
   <211> 24
   <212> PRT
   <213> Artificial
<220>
   <223> künstliche Sekretionssequenz
<400> 41
   Met Lys Arg Leu Cys Leu Trp Phe Thr Val Phe Ser Leu Phe Leu Val
   1               5                   10                  15
   Leu Leu Pro Gly Lys Ala Leu Gly
               20
<210> 42
   <211> 36
   <212> PRT
   <213> Artificial
<220>
   <223> künstliche Sekretionssequenz
<400> 42
   Met Lys Leu Lys Thr Lys Ala Ser Ile Lys Phe Gly Ile Cys Val Gly
   1               5                   10                  15
   Leu Leu Cys Leu Ser Ile Thr Gly Phe Thr Pro Phe Phe Asn Ser Thr
               20                  25                  30
   His Ala Glu Ala
           35
<210> 43
   <211> 31
   <212> PRT
   <213> Artificial
<220>
   <223> künstliche Sekretionssequenz
<400> 43
   Met Lys Lys Met Ser Leu Phe Gln Asn Met Lys Ser Lys Leu Leu Pro
   1               5                   10                  15
   Ile Ala Ala Val Ser Val Leu Thr Ala Gly Ile Phe Ala Gly Ala
               20                  25                  30
<210> 44
   <211> 32
   <212> PRT
   <213> Artificial
<220>
   <223> künstliche Sekretionssequenz
<400> 44
   Met Lys Lys Phe Pro Lys Lys Leu Leu Pro Ile Ala Val Leu Ser Ser
   1               5                   10                  15
   Ile Ala Phe Ser Ser Leu Ala Ser Gly Ser Val Pro Glu Ala Ser Ala
               20                  25                  30
<210> 45
   <211> 25
   <212> PRT
   <213> Artificial
<220>
   <223> künstliche Sekretionssequenz
<400> 45
   Met Lys Ser Lys Trp Met Ser Gly Leu Leu Leu Val Ala Val Gly Phe
   1               5                   10                  15
   Ser Phe Thr Gln Val Met Val His Ala
               20                  25
<210> 46
   <211> 27
   <212> PRT
   <213> Artificial
<220>
   <223> künstliche Sekretionssequenz
<400> 46
   Met Lys Leu Lys Ser Lys Leu Phe Val Ile Cys Leu Ala Ala Ala Ala
   1               5                   10                  15
   Ile Phe Thr Ala Ala Gly Val Ser Ala Asn Ala
               20                  25
<210> 47
   <211> 24
   <212> PRT
   <213> Artificial
<220>
   <223> künstliche Sekretionssequenz
<400> 47
   Met Lys Leu Lys Ser Lys Leu Leu Leu Ser Cys Leu Ala Leu Ser Thr
   1               5                   10                  15
   Val Phe Val Ala Thr Thr Ile Ala
               20
<210> 48
   <211> 21
   <212> PRT
   <213> Artificial
<220>
   <223> künstliche Sekretionssequenz
<400> 48
   Met Lys Arg Phe Leu Ile Gly Ala Gly Val Ala Ala Val Ile Leu Ser
   1               5                   10                  15
   Gly Trp Phe Ile Ala
               20
<210> 49
   <211> 19
   <212> PRT
   <213> Artificial
<220>
   <223> künstliche Sekretionssequenz
<400> 49
   Met Lys Lys Leu Val Leu Cys Val Ser Ile Leu Ala Val Ile Leu Ser
   1               5                   10                  15
   Gly Val Ala
<210> 50
   <211> 26
   <212> PRT
   <213> Artificial
<220>
   <223> künstliche Sekretionssequenz
<400> 50
   Met Arg Lys Lys Ile Thr Leu Ala Cys Lys Thr Cys Gly Asn Arg Asn
   1               5                   10                  15
   Tyr Thr Thr Met Lys Ser Ser Ala Ser Ala
               20                  25
<210> 51
   <211> 29
   <212> PRT
   <213> Artificial
<220>
   <223> künstliche Sekretionssequenz
<400> 51
   Met Asn Ile Lys Lys Phe Ala Lys Gln Ala Thr Val Leu Thr Phe Thr
   1               5                   10                  15
   Thr Ala Leu Leu Ala Gly Gly Ala Thr Gln Ala Phe Ala
               20                  25
<210> 52
   <211> 24
   <212> PRT
   <213> Artificial
<220>
   <223> künstliche Sekretionssequenz
<400> 52
   Met Lys Lys Arg Leu Ile Gln Val Met Ile Met Phe Thr Leu Leu Leu
   1               5                   10                  15
   Thr Met Ala Phe Ser Ala Asp Ala
               20
<210> 53
   <211> 31
   <212> PRT
   <213> Artificial
<220>
   <223> künstliche Sekretionssequenz
<400> 53
   Met Lys Ser Lys Gly Ser Ile Met Ala Cys Leu Ile Leu Phe Ser Phe
   1               5                   10                  15
   Thr Ile Thr Thr Phe Ile Asn Thr Glu Thr Ile Ser Ala Phe Ser
               20                  25                  30
<210> 54
   <211> 34
   <212> PRT
   <213> Artificial
<220>
   <223> künstliche Sekretionssequenz
<400> 54
   Met Lys Gln Phe Ala Ile Thr Leu Ser Val Leu Cys Ala Leu Ile Leu
   1               5                   10                  15
   Leu Val Pro Thr Leu Leu Val Ile Pro Phe Gln His Asn Lys Glu Ala
               20                  25                  30
   Gly Ala
<210> 55
   <211> 39
   <212> PRT
   <213> Artificial
<220>
   <223> künstliche Sekretionssequenz
<400> 55
   Met Arg Asn Lys Arg Arg Asn Arg Gln Ile Val Val Ala Val Asn Gly
   1               5                   10                  15
   Gly Lys Ala Val Lys Ala Ile Phe Leu Phe Ile Val Ser Leu Ile Val
               20                  25                  30
   Ile Phe Val Leu Ser Gly Val
           35
<210> 56
   <211> 40
   <212> PRT
   <213> Artificial
<220>
   <223> künstliche Sekretionssequenz
<400> 56
   Met Arg Glu Glu Glu Lys Lys Thr Ser Gln Val Lys Lys Leu Gln Gln
   1               5                   10                  15
   Phe Phe Arg Lys Arg Trp Val Phe Pro Ala Ile Tyr Leu Val Ser Ala
               20                  25                  30
   Ala Val Ile Leu Thr Ala Val Leu
           35                  40
<210> 57
   <211> 20
   <212> PRT
   <213> Artificial
<220>
   <223> künstliche Sekretionssequenz
<400> 57
   Met Lys Lys Thr Val Ile Ile Cys Ile Tyr Ile Phe Leu Leu Leu Ser
   1               5                   10                  15
   Gly Ala Leu Val
               20
<210> 58
   <211> 27
   <212> PRT
   <213> Artificial
<220>
   <223> künstliche Sekretionssequenz
<400> 58
   Met Gly Met Lys Lys Lys Leu Ser Leu Gly Val Ala Ser Ala Ala Leu
   1               5                   10                  15
   Gly Leu Ala Leu Val Gly Gly Gly Thr Trp Ala
               20                  25
<210> 59
   <211> 23
   <212> PRT
   <213> Artificial
<220>
   <223> künstliche Sekretionssequenz
<400> 59
   Met Asn Gln Met Lys Asp Thr Ile Leu Leu Ala Gly Leu Gly Leu Ile
   1               5                   10                  15
   Gly Gly Ser Ile Ala Leu Ala
               20
<210> 60
   <211> 31
   <212> PRT
   <213> Artificial
<220>
   <223> künstliche Sekretionssequenz
<400> 60
   Met Lys Lys Gly Ile Ile Arg Phe Leu Leu Val Ser Phe Val Leu Phe
   1 5 10 15
   Phe Ala Leu Ser Thr Gly Ile Thr Gly Val Gln Ala Ala Pro Ala
   20 25 30
<210> 61
   <211> 28
   <212> PRT
   <213> Artificial
<220>
   <223> künstliche Sekretionssequenz
<400> 61
   Met Phe Lys Phe Lys Lys Asn Phe Leu Val Gly Leu Ser Ala Ala Leu
   1 5 10 15
   Met Ser Ile Ser Leu Phe Ser Ala Thr Ala Ser Ala
   20 25
<210> 62
   <211> 23
   <212> PRT
   <213> Artificial
<220>
   <223> künstliche Sekretionssequenz
<400> 62
   Met Arg Lys Thr Ile Phe Ala Phe Leu Thr Gly Leu Met Met Phe Gly
   1 5 10 15
   Thr Ile Thr Ala Ala Ser Ala
   20
<210> 63
   <211> 25
   <212> PRT
   <213> Artificial
<220>
   <223> künstliche Sekretionssequenz
<400> 63
   Met Lys Thr Lys Thr Leu Phe Ile Phe Ser Ala Ile Leu Thr Leu Ser
   1 5 10 15
   Ile Phe Ala Pro Asn Glu Thr Phe Ala
   20 25
<210> 64
   <211> 28
   <212> PRT
   <213> Artificial
<220>
   <223> künstliche Sekretionssequenz
<400> 64
   Met Asp Lys Phe Leu Asn Asn Arg Trp Ala Val Lys Ile Ile Ala Leu
   1               5                   10                  15
   Leu Phe Ala Leu Leu Leu Tyr Val Ala Val Asn Ser
               20                  25
<210> 65
   <211> 25
   <212> PRT
   <213> Artificial
<220>
   <223> künstliche Sekretionssequenz
<400> 65
   Met Lys Thr Leu Trp Lys Val Leu Lys Ile Val Phe Val Ser Leu Ala
   1               5                   10                  15
   Ala Leu Val Leu Leu Val Ser Val Ser
               20                  25
<210> 66
   <211> 25
   <212> PRT
   <213> Artificial
<220>
   <223> künstliche Sekretionssequenz
<400> 66
   Met Val Lys Lys Trp Leu Ile Gln Phe Ala Val Met Leu Ser Val Leu
   1               5                   10                  15
   Ser Thr Phe Thr Tyr Ser Ala Ser Ala
               20                  25
<210> 67
   <211> 28
   <212> PRT
   <213> Artificial
<220>
   <223> künstliche Sekretionssequenz
<400> 67
   Met Lys Arg Met Ile Val Arg Met Thr Leu Pro Leu Leu Ile Val Cys
   1               5                   10                  15
   Leu Ala Phe Ser Ser Phe Ser Ala Ser Ala Arg Ala
               20                  25
<210> 68
   <211> 23
   <212> PRT
   <213> Artificial
<220>
   <223> künstliche Sekretionssequenz
<400> 68
   Met Lys Lys Trp Ile Tyr Val Val Leu Val Leu Ser Ile Ala Gly Ile
   1               5                   10                  15
   Gly Gly Phe Ser Val His Ala
               20
<210> 69
   <211> 26
   <212> PRT
   <213> Artificial
<220>
   <223> künstliche Sekretionssequenz
<400> 69
   Met Lys Arg Ile Thr Ile Asn Ile Ile Thr Met Phe Ile Ala Ala Ala
   1               5                   10                  15
   Val Ile Ser Leu Thr Gly Thr Ala Glu Ala
               20                  25
<210> 70
   <211> 32
   <212> PRT
   <213> Artificial
<220>
   <223> künstliche Sekretionssequenz
<400> 70
   Met Lys Leu Phe Asn Arg Lys Val Thr Leu Val Ser Leu Ile Leu Met
   1               5                   10                  15
   Ala Val Phe Gln Phe Phe Met Ala Leu Ile Lys Arg Ile Val Ile Ser
               20                  25                  30
<210> 71
   <211> 28
   <212> PRT
   <213> Artificial
<220>
   <223> künstliche Sekretionssequenz
<400> 71
   Met Arg Lys Lys Arg Val Ile Thr Cys Val Met Ala Ala Ser Leu Thr
   1               5                   10                  15
   Leu Gly Ser Leu Leu Pro Ala Gly Tyr Ala Ser Ala
               20                  25
<210> 72
   <211> 21
   <212> PRT
   <213> Artificial
<220>
   <223> künstliche Sekretionssequenz
<400> 72
   Met Phe Lys Lys His Thr Ile Ser Leu Leu Ile Ile Phe Leu Leu Ala
   1               5                   10                  15
   Ser Ala Val Leu Ala
               20
<210> 73
   <211> 28
   <212> PRT
   <213> Artificial
<220>
   <223> künstliche Sekretionssequenz
<400> 73
   Met Leu Lys Lys Val Ile Leu Ala Ala Phe Ile Leu Val Gly Ser Thr
   1               5                   10                  15
   Leu Gly Ala Phe Ser Phe Ser Ser Asp Ala Ser Ala
               20                  25
<210> 74
   <211> 21
   <212> PRT
   <213> Artificial
<220>
   <223> künstliche Sekretionssequenz
<400> 74
   Met Lys Lys Arg Ile Ile Leu Leu Leu Ala Val Ile Ile Ala Ala Ala
   1               5                   10                  15
   Ala Ala Gly Val Ala
               20
<210> 75
   <211> 29
   <212> PRT
   <213> Artificial
<220>
   <223> künstliche Sekretionssequenz
<400> 75
   Met Lys Lys Lys Gln Val Met Leu Ala Leu Thr Ala Ala Ala Gly Leu
   1               5                   10                  15
   Gly Leu Thr Ala Leu His Ser Ala Pro Ala Ala Lys Ala
               20                  25
<210> 76
   <211> 21
   <212> PRT
   <213> Artificial
<220>
   <223> künstliche Sekretionssequenz
<400> 76
   Met Lys Trp Met Cys Ser Ile Cys Cys Ala Ala Val Leu Leu Ala Gly
   1               5                   10                  15
   Gly Ala Ala Gln Ala
               20
<210> 77
   <211> 26
   <212> PRT
   <213> Artificial
<220>
   <223> künstliche Sekretionssequenz
<400> 77
   Met Met Lys Lys Leu Phe His Ser Thr Leu Ile Val Leu Leu Phe Phe
   1               5                   10                  15
   Ser Phe Phe Gly Val Gln Pro Ile His Ala
               20                  25
<210> 78
   <211> 34
   <212> PRT
   <213> Artificial
<220>
   <223> künstliche Sekretionssequenz
<400> 78
   Met Arg Thr Trp Lys Arg Ile Pro Lys Thr Thr Met Leu Ile Ser Leu
   1               5                   10                  15
   Val Ser Pro Phe Leu Leu Ile Thr Pro Val Leu Phe Tyr Ala Ala Leu
               20                  25                  30
   Ala Phe
<210> 79
   <211> 21
   <212> PRT
   <213> Artificial
<220>
   <223> künstliche Sekretionssequenz
<400> 79
   Met Lys Ser Leu Pro Tyr Thr Ile Ala Leu Leu Phe Cys Gly Leu Ile
   1               5                   10                  15
   Ile Val Ser Met Ala
               20
<210> 80
   <211> 36
   <212> PRT
   <213> Artificial
<220>
   <223> künstliche Sekretionssequenz
<400> 80
   Met Lys Lys Ile Val Ala Ala Ile Val Val Ile Gly Leu Val Phe Ile
   1               5                   10                  15
   Ala Phe Phe Tyr Leu Tyr Ser Arg Ser Gly Asp Val Tyr Gln Ser Val
               20                  25                  30
   Asp Ala Asp Leu
               35
<210> 81
   <211> 27
   <212> PRT
   <213> Artificial
<220>
   <223> künstliche Sekretionssequenz
<400> 81
   Met Lys Lys Ala Ala Ala Val Leu Leu Ser Leu Gly Leu Val Phe Gly
   1               5                   10                  15
   Phe Ser Tyr Gly Ala Gly His Val Ala Glu Ala
               20                  25
<210> 82
   <211> 26
   <212> PRT
   <213> Artificial
<220>
   <223> künstliche Sekretionssequenz
<400> 82
   Met Lys Lys Glu Leu Leu Ala Ser Leu Val Leu Cys Leu Ser Leu Ser
   1               5                   10                  15
   Pro Leu Val Ser Thr Asn Glu Val Phe Ala
               20                  25
<210> 83
   <211> 34
   <212> PRT
   <213> Artificial
<220>
   <223> künstliche Sekretionssequenz
<400> 83
   Met Lys Lys Lys Thr Lys Ile Ile Leu Ser Leu Leu Ala Ala Leu Ile
   1               5                   10                  15
   Val Ile Leu Ile Val Leu Pro Val Leu Ser Pro Val Val Phe Thr Ala
               20                  25                  30
   Ser Ser
<210> 84
   <211> 26
   <212> PRT
   <213> Artificial
<220>
   <223> künstliche Sekretionssequenz
<400> 84
   Met Asn Phe Lys Lys Thr Val Val Ser Ala Leu Ser Ile Ser Ala Leu
   1               5                   10                  15
   Ala Leu Ser Val Ser Gly Val Ala Ser Ala
               20                  25
<210> 85
   <211> 28
   <212> PRT
   <213> Artificial
<220>
   <223> künstliche Sekretionssequenz
<400> 85
   Met Lys Arg Leu Phe Met Lys Ala Ser Leu Val Leu Phe Ala Val Val
   1               5                   10                  15
   Phe Val Phe Ala Val Lys Gly Ala Pro Ala Lys Ala
               20                  25
<210> 86
   <211> 26
   <212> PRT
   <213> Artificial
<220>
   <223> künstliche Sekretionssequenz
<400> 86
   Met Ala Ala Gln Thr Asp Tyr Lys Lys Gln Val Val Gly Ile Leu Leu
   1               5                   10                  15
   Ser Leu Ala Phe Val Leu Phe Val Phe Ser
               20                  25
<210> 87
   <211> 33
   <212> PRT
   <213> Artificial
<220>
   <223> künstliche Sekretionssequenz
<400> 87
   Met Lys Lys Val Trp Ile Gly Ile Gly Ile Ala Val Ile Val Ala Leu
   1               5                   10                  15
   Phe Val Gly Ile Asn Ile Tyr Arg Ser Ala Ala Pro Thr Ser Gly Ser
               20                  25                  30
   Ala
<210> 88
   <211> 26
   <212> PRT
   <213> Artificial
<220>
   <223> künstliche Sekretionssequenz
<400> 88
   Met Leu Lys Lys Lys Trp Met Val Gly Leu Leu Ala Gly Cys Leu Ala
   1               5                   10                  15
   Ala Gly Gly Phe Ser Tyr Asn Ala Phe Ala
               20                  25
<210> 89
   <211> 21
   <212> PRT
   <213> Artificial
<220>
   <223> künstliche Sekretionssequenz
<400> 89
   Met Thr Thr Lys Phe Thr Ala Leu Ala Val Phe Leu Leu Cys Phe Met
   1               5                   10                  15
   Pro Ala Ala Lys Ile
               20
<210> 90
   <211> 26
   <212> PRT
   <213> Artificial
<220>
   <223> künstliche Sekretionssequenz
<400> 90
   Met Leu Thr Lys Arg Leu Leu Thr Ile Tyr Ile Met Leu Leu Gly Leu
   1               5                   10                  15
   Ile Ala Trp Phe Pro Gly Ala Ala Gln Ala
               20                  25
<210> 91
   <211> 26
   <212> PRT
   <213> Artificial
<220>
   <223> künstliche Sekretionssequenz
<400> 91
   Met Lys Lys Ala Phe Ile Leu Ser Ala Ala Ala Ala Val Gly Leu Phe
   1               5                   10                  15
   Thr Phe Gly Gly Val Gln Gln Ala Ser Ala
               20                  25
<210> 92
   <211> 31
   <212> PRT
   <213> Artificial
<220>
   <223> künstliche Sekretionssequenz
<400> 92
   Met Lys Lys Thr Phe Val Lys Lys Ala Met Leu Thr Thr Ala Ala Met
   1               5                   10                  15
   Thr Ser Ala Ala Leu Leu Thr Phe Gly Pro Asp Ala Ala Ser Ala
               20                  25                  30
<210> 93
   <211> 35
   <212> PRT
   <213> Artificial
<220>
   <223> künstliche Sekretionssequenz
<400> 93
   Met Leu Arg Lys Lys His Phe Ser Trp Met Leu Val Ile Leu Ile Leu
   1               5                   10                  15
   Ile Ala Val Leu Ser Phe Ile Lys Leu Pro Tyr Tyr Ile Thr Lys Pro
               20                  25                  30
   Gly Glu Ala
           35
<210> 94
   <211> 27
   <212> PRT
   <213> Artificial
<220>
   <223> künstliche Sekretionssequenz
<400> 94
   Met Lys Lys Ile Gly Leu Leu Phe Met Leu Cys Leu Ala Ala Leu Phe
   1               5                   10                  15
   Thr Ile Gly Phe Pro Ala Gln Gln Ala Asp Ala
               20                  25
<210> 95
   <211> 37
   <212> PRT
   <213> Artificial
<220>
   <223> künstliche Sekretionssequenz
<400> 95
   Met Ser Gly Lys Lys Lys Glu Ser Gly Lys Phe Arg Ser Val Leu Leu
   1               5                   10                  15
   Ile Ile Ile Leu Pro Leu Met Phe Leu Leu Ile Ala Gly Gly Ile Val
               20                  25                  30
   Leu Trp Ala Ala Gly
           35
<210> 96
   <211> 35
   <212> PRT
   <213> Artificial
<220>
   <223> künstliche Sekretionssequenz
<400> 96
   Met Arg Gly Lys Ser Ala Val Leu Leu Ser Leu Ile Met Leu Ile Ala
   1               5                   10                  15
   Gly Phe Leu Ile Ser Phe Ser Phe Gln Met Thr Lys Glu Asn Asn Lys
               20                  25                  30
   Ser Ala Ala
           35
<210> 97
   <211> 33
   <212> PRT
   <213> Artificial
<220>
   <223> künstliche Sekretionssequenz
<400> 97
   Met Tyr Lys Lys Phe Val Pro Phe Ala Val Phe Leu Phe Leu Phe Phe
   1               5                   10                  15
   Val Ser Phe Glu Met Met Glu Asn Pro His Ala Leu Asp Tyr Ile Gly
               20                  25                  30
   Ala
<210> 98
   <211> 42
   <212> PRT
   <213> Artificial
<220>
   <223> künstliche Sekretionssequenz
<400> 98
   Met Ala Lys Pro Leu Ser Lys Gly Gly Ile Leu Val Lys Lys Val Leu
   1               5                   10                  15
   Ile Ala Gly Ala Val Gly Thr Ala Val Leu Phe Gly Thr Leu Ser Ser
               20                  25                  30
   Gly Ile Pro Gly Leu Pro Ala Ala Asp Ala
           35                  40
<210> 99
   <211> 25
   <212> PRT
   <213> Artificial
<220>
   <223> künstliche Sekretionssequenz
<400> 99
   Met Arg Phe Thr Lys Val Val Gly Phe Leu Ser Val Leu Gly Leu Ala
   1               5                   10                  15
   Ala Val Phe Pro Leu Thr Ala Gln Ala
               20                  25
<210> 100
   <211> 32
   <212> PRT
   <213> Artificial
<220>
   <223> künstliche Sekretionssequenz
<400> 100
   Met Ile Pro Arg Ile Lys Lys Thr Ile Cys Val Leu Leu Val Cys Phe
   1               5                   10                  15
   Thr Met Leu Ser Val Met Leu Gly Pro Gly Ala Thr Glu Val Leu Ala
               20                  25                  30
<210> 101
   <211> 30
   <212> PRT
   <213> Artificial
<220>
   <223> künstliche Sekretionssequenz
<400> 101
   Met Lys Val Cys Gln Lys Ser Ile Val Arg Phe Leu Val Ser Leu Ile
   1               5                   10                  15
   Ile Gly Thr Phe Val Ile Ser Val Pro Phe Met Ala Asn Ala
               20                  25                  30
<210> 102
   <211> 28
   <212> PRT
   <213> Artificial
<220>
   <223> künstliche Sekretionssequenz
<400> 102
   Met Glu Leu Ser Phe Thr Lys Ile Leu Val Ile Leu Phe Val Gly Phe
   1               5                   10                  15
   Leu Val Phe Gly Pro Asp Lys Leu Pro Ala Leu Gly
               20                  25
<210> 103
   <211> 24
   <212> PRT
   <213> Artificial
<220>
   <223> künstliche Sekretionssequenz
<400> 103
   Met Lys Lys Met Leu Met Leu Ala Phe Thr Phe Leu Leu Ala Leu Thr
   1               5                   10                  15
   Ile His Val Gly Glu Ala Ser Ala
               20
<210> 104
   <211> 25
   <212> PRT
   <213> Artificial
<220>
   <223> künstliche Sekretionssequenz
<400> 104
   Met Lys Ile Arg Lys Ile Leu Leu Ser Ser Ala Leu Ser Phe Gly Met
   1               5                   10                  15
   Leu Ile Ser Ala Val Pro Ala Leu Ala
               20                  25
<210> 105
   <211> 21
   <212> PRT
   <213> Artificial
<220>
   <223> künstliche Sekretionssequenz
<400> 105
   Met Lys Leu Glu Arg Leu Leu Ala Met Val Val Leu Leu Ile Ser Lys
   1               5                   10                  15
   Lys Gln Val Gln Ala
               20
<210> 106
   <211> 23
   <212> PRT
   <213> Artificial
<220>
   <223> künstliche Sekretionssequenz
<400> 106
   Met Lys Lys Lys Arg Lys Gly Cys Phe Ala Ala Ala Gly Phe Met Met
   1               5                   10                  15
   Ile Phe Val Phe Val Ile Ala
               20
<210> 107
   <211> 23
   <212> PRT
   <213> Artificial
<220>
   <223> künstliche Sekretionssequenz
<400> 107
   Met Lys Lys Thr Ile Met Ser Phe Val Ala Val Ala Ala Leu Ser Thr
   1               5                   10                  15
   Thr Ala Phe Gly Ala His Ala
               20
<210> 108
   <211> 26
   <212> PRT
   <213> Artificial
<220>
   <223> künstliche Sekretionssequenz
<400> 108
   Met Lys Val Pro Lys Thr Met Leu Leu Ser Thr Ala Ala Gly Leu Leu
   1               5                   10                  15
   Leu Ser Leu Thr Ala Thr Ser Val Ser Ala
               20                  25
<210> 109
   <211> 26
   <212> PRT
   <213> Artificial
<220>
   <223> künstliche Sekretionssequenz
<400> 109
   Met Lys Lys Lys Ile Val Ala Gly Leu Ala Val Ser Ala Val Val Gly
   1               5                   10                  15
   Ser Ser Met Ala Ala Ala Pro Ala Glu Ala
               20                  25
<210> 110
   <211> 28
   <212> PRT
   <213> Artificial
<220>
   <223> künstliche Sekretionssequenz
<400> 110
   Met Lys Lys Arg Ile Thr Tyr Ser Leu Leu Ala Leu Leu Ala Val Val
   1               5                   10                  15
   Ala Phe Ala Phe Thr Asp Ser Ser Lys Ala Lys Ala
               20                  25
<210> 111
   <211> 25
   <212> PRT
   <213> Artificial
<220>
   <223> künstliche Sekretionssequenz
<400> 111
   Met Lys Lys Arg Leu Ile Gly Phe Leu Val Leu Val Pro Ala Leu Ile
   1               5                   10                  15
   Met Ser Gly Ile Thr Leu Ile Glu Ala
               20                  25
<210> 112
   <211> 27
   <212> PRT
   <213> Artificial
<220>
   <223> künstliche Sekretionssequenz
<400> 112
   Met Lys Lys Trp Ile Val Leu Phe Leu Val Leu Ile Ala Ala Ala Ile
   1               5                   10                  15
   Ser Ile Phe Val Tyr Val Ser Thr Gly Ser Glu
               20                  25
<210> 113
   <211> 28
   <212> PRT
   <213> Artificial
<220>
   <223> künstliche Sekretionssequenz
<400> 113
   Met Arg Lys Lys Arg Val Ile Thr Cys Val Met Ala Ala Ser Leu Thr
   1               5                   10                  15
   Leu Gly Ser Leu Leu Pro Ala Gly Tyr Ala Thr Ala
               20                  25
<210> 114
   <211> 21
   <212> PRT
   <213> Artificial
<220>
   <223> künstliche Sekretionssequenz
<400> 114
   Met Lys Lys Leu Ile Met Ala Leu Val Ile Leu Gly Ala Leu Gly Thr
   1               5                   10                  15
   Ser Tyr Ile Ser Ala
               20
<210> 115
   <211> 23
   <212> PRT
   <213> Artificial
<220>
   <223> künstliche Sekretionssequenz
<400> 115
   Met Lys Arg Phe Ile Leu Val Leu Ser Phe Leu Ser Ile Ile Val Ala
   1               5                   10                  15
   Tyr Pro Ile Gln Thr Asn Ala
               20
<210> 116
   <211> 25
   <212> PRT
   <213> Artificial
<220>
   <223> künstliche Sekretionssequenz
<400> 116
   Met Lys Leu Arg Lys Val Leu Thr Gly Ser Val Leu Ser Leu Gly Leu
   1               5                   10                  15
   Leu Val Ser Ala Ser Pro Ala Phe Ala
               20                  25
<210> 117
   <211> 26
   <212> PRT
   <213> Artificial
<220>
   <223> künstliche Sekretionssequenz
<400> 117
   Met Lys Leu Ser Val Lys Ile Ala Gly Val Leu Thr Val Ala Ala Ala
   1               5                   10                  15
   Ala Met Thr Ala Lys Met Tyr Ala Thr Ala
               20                  25
<210> 118
   <211> 23
   <212> PRT
   <213> Artificial
<220>
   <223> künstliche Sekretionssequenz
<400> 118
   Met Asn Asn Asn Lys Leu Leu Leu Val Asp Gly Met Ala Leu Leu Phe
   1               5                   10                  15
   Arg Ala Phe Phe Ala Thr Ala
               20
<210> 119
   <211> 29
   <212> PRT
   <213> Artificial
<220>
   <223> künstliche Sekretionssequenz
<400> 119
   Met Lys Leu Trp Met Arg Lys Thr Leu Val Val Leu Phe Thr Ile Val
   1               5                   10                  15
   Thr Phe Gly Leu Val Ser Pro Pro Ala Ala Leu Met Ala
               20                  25
<210> 120
   <211> 32
   <212> PRT
   <213> Artificial
<220>
   <223> künstliche Sekretionssequenz
<400> 120
   Met Arg Lys Lys Ala Leu Ile Phe Thr Val Ile Phe Gly Ile Ile Phe
   1               5                   10                  15
   Leu Ala Val Leu Leu Val Ser Ala Ser Ile Tyr Lys Ser Ala Met Ala
               20                  25                  30
<210> 121
   <211> 35
   <212> PRT
   <213> Artificial
<220>
   <223> künstliche Sekretionssequenz
<400> 121
   Met Asn Lys Trp Lys Arg Leu Phe Phe Ile Leu Leu Ala Ile Asn Phe
   1               5                   10                  15
   Ile Leu Ala Ala Gly Phe Val Ala Leu Val Leu Leu Pro Gly Glu Gln
               20                  25                  30
   Ala Gln Val
           35
<210> 122
   <211> 26
   <212> PRT
   <213> Artificial
<220>
   <223> künstliche Sekretionssequenz
<400> 122
   Met Lys Lys Ile Trp Ile Gly Met Leu Ala Ala Ala Val Leu Leu Leu
   1               5                   10                  15
   Met Val Pro Lys Val Ser Leu Ala Asp Ala
               20                  25
<210> 123
   <211> 38
   <212> PRT
   <213> Artificial
<220>
   <223> künstliche Sekretionssequenz
<400> 123
   Met Gly Arg Ile Lys Thr Lys Ile Thr Ile Leu Leu Val Leu Leu Leu
   1               5                   10                  15
   Leu Leu Ala Gly Gly Tyr Met Tyr Ile Asn Asp Ile Glu Leu Lys Asp
               20                  25                  30
   Val Pro Thr Ala Ile Gly
           35
<210> 124
   <211> 38
   <212> PRT
   <213> Artificial
<220>
   <223> künstliche Sekretionssequenz
<400> 124
   Met Lys Arg Leu Thr Leu Val Cys Ser Thr Val Phe Ile Leu Phe Ile
   1               5                   10                  15
   Leu Phe Tyr Asp Leu Lys Ile Gly Thr Ile Pro Ile Gln Asp Leu Pro
               20                  25                  30
   Val Tyr Glu Ala Ser Ala
           35
<210> 125
   <211> 28
   <212> PRT
   <213> Artificial
<220>
   <223> künstliche Sekretionssequenz
<400> 125
   Met Lys Gln Gly Lys Phe Ser Val Phe Leu Ile Leu Leu Leu Met Leu
   1               5                   10                  15
   Thr Leu Val Val Ala Pro Lys Gly Lys Ala Glu Ala
               20                  25
<210> 126
   <211> 28
   <212> PRT
   <213> Artificial
<220>
   <223> künstliche Sekretionssequenz
<400> 126
   Met Phe Lys Lys Leu Leu Leu Ala Thr Ser Ala Leu Thr Phe Ser Leu
   1               5                   10                  15
   Ser Leu Val Leu Pro Leu Asp Gly His Ala Lys Ala
               20                  25
<210> 127
   <211> 43
   <212> PRT
   <213> Artificial
<220>
   <223> künstliche Sekretionssequenz
<400> 127
   Met Phe Arg Leu Phe His Asn Gln Gln Lys Ala Lys Thr Lys Leu Lys
   1 5 10 15
   Val Leu Leu Ile Phe Gln Leu Ser Val Ile Phe Ser Leu Thr Ala Ala
   20 25 30
   Ile Cys Leu Gln Phe Ser Asp Asp Thr Ser Ala
   35 40
<210> 128
   <211> 34
   <212> PRT
   <213> Artificial
<220>
   <223> künstliche Sekretionssequenz
<400> 128
   Met Thr Lys Arg Gly Ile Gln Ala Phe Ala Gly Gly Ile Ile Leu Ala
   1 5 10 15
   Thr Ala Val Leu Ala Ala Val Phe Tyr Leu Thr Asp Glu Asp Gln Ala
   20 25 30
   Ala Ala
<210> 129
   <211> 22
   <212> PRT
   <213> Artificial
<220>
   <223> künstliche Sekretionssequenz
<400> 129
   Met Met Ile Lys Gln Cys Val Ile Cys Leu Ser Leu Leu Val Phe Gly
   1 5 10 15
   Thr Thr Ala Ala His Ala
   20
<210> 130
   <211> 28
   <212> PRT
   <213> Artificial
<220>
   <223> künstliche Sekretionssequenz
<400> 130
   Met Thr Leu Thr Lys Leu Lys Met Leu Ser Met Leu Thr Val Met Ile
   1               5                   10                  15
   Ala Ser Leu Phe Ile Phe Ser Ser Gln Ala Leu Ala
               20                  25
<210> 131
   <211> 33
   <212> PRT
   <213> Artificial
<220>
   <223> künstliche Sekretionssequenz
<400> 131
   Met Tyr Ile Asn Gln Gln Lys Lys Ser Phe Phe Asn Lys Lys Arg Ile
   1               5                   10                  15
   Ile Leu Ser Ser Ile Val Val Leu Phe Leu Thr Ile Gly Gly Ala Phe
               20                  25                  30
   Leu
<210> 132
   <211> 27
   <212> PRT
   <213> Artificial
<220>
   <223> künstliche Sekretionssequenz
<400> 132
   Met Lys Ile Ser Lys Arg Met Lys Leu Ala Val Ile Ala Phe Leu Ile
   1               5                   10                  15
   Val Phe Phe Leu Leu Leu Arg Leu Ala Glu Ile
               20                  25
<210> 133
   <211> 37
   <212> PRT
   <213> Artificial
<220>
   <223> künstliche Sekretionssequenz
<400> 133
   Met Ser Asn Asn Gln Ser Arg Tyr Glu Asn Arg Asp Lys Arg Arg Lys
   1               5                   10                  15
   Ala Asn Leu Val Leu Asn Ile Leu Ile Ala Ile Val Ser Ile Leu Ile
               20                  25                  30
   Val Val Val Ala Ala
           35
<210> 134
   <211> 27
   <212> PRT
   <213> Artificial
<220>
   <223> künstliche Sekretionssequenz
<400> 134
   Met Asn Lys Lys Tyr Phe Val Leu Ile Val Cys Ile Ile Phe Thr Ser
   1               5                   10                  15
   Ala Leu Phe Pro Thr Phe Ser Ser Val Thr Ala
               20                  25
<210> 135
   <211> 30
   <212> PRT
   <213> Artificial
<220>
   <223> künstliche Sekretionssequenz
<400> 135
   Met Lys Arg Lys Leu Leu Ser Ser Leu Ala Ile Ser Ala Leu Ser Leu
   1               5                   10                  15
   Gly Leu Leu Val Ser Ala Pro Thr Ala Ser Phe Ala Ala Glu
               20                  25                  30
<210> 136
   <211> 37
   <212> PRT
   <213> Artificial
<220>
   <223> künstliche Sekretionssequenz
<400> 136
   Met Ile Thr Asp Ile Phe Lys Pro Gly Cys Arg Lys Leu Cys Val Phe
   1 5 10 15
   Asn Met Lys Gly Asp Tyr Phe Val Lys Val Leu Leu Ser Ala Leu Leu
   20 25 30
   Leu Leu Leu Phe Ala
   35
<210> 137
   <211> 35
   <212> PRT
   <213> Artificial
<220>
   <223> künstliche Sekretionssequenz
<400> 137
   Met Pro Arg Tyr Arg Gly Pro Phe Arg Lys Arg Gly Pro Leu Pro Phe
   1 5 10 15
   Arg Tyr Val Met Leu Leu Ser Val Val Phe Phe Ile Leu Ser Thr Thr
   20 25 30
   Val Ser Leu
   35
<210> 138
   <211> 28
   <212> PRT
   <213> Artificial
<220>
   <223> künstliche Sekretionssequenz
<400> 138
   Met Thr Lys Lys Ala Trp Phe Leu Pro Leu Val Cys Val Leu Leu Ile
   1 5 10 15
   Ser Gly Trp Leu Ala Pro Ala Ala Ser Ala Ser Ala
   20 25
<210> 139
   <211> 31
   <212> PRT
   <213> Artificial
<220>
   <223> künstliche Sekretionssequenz
<400> 139
   Met His Leu Ile Arg Ala Ala Gly Ala Val Cys Leu Ala Val Val Leu
   1               5                   10                  15
   Ile Ala Gly Cys Arg Phe Asn Glu Asp Gln His Gln Ala Glu Gly
               20                  25                  30
<210> 140
   <211> 27
   <212> PRT
   <213> Artificial
<220>
   <223> künstliche Sekretionssequenz
<400> 140
   Met Lys Lys Trp Leu Ile Ile Ala Val Ser Leu Ala Ile Ala Ile Val
   1               5                   10                  15
   Leu Phe Met Tyr Thr Lys Gly Glu Ala Lys Ala
               20                  25
<210> 141
   <211> 30
   <212> PRT
   <213> Artificial
<220>
   <223> künstliche Sekretionssequenz
<400> 141
   Met Arg Lys Ser Leu Ile Thr Leu Gly Leu Ala Ser Val Ile Gly Thr
   1               5                   10                  15
   Ser Ser Phe Leu Ile Pro Phe Thr Ser Lys Thr Ala Ser Ala
               20                  25                  30
<210> 142
   <211> 25
   <212> PRT
   <213> Artificial
<220>
   <223> künstliche Sekretionssequenz
<400> 142
   Met Asn Tyr Ile Lys Ala Gly Lys Trp Leu Thr Val Phe Leu Thr Phe
   1               5                   10                  15
   Leu Gly Ile Leu Leu Phe Ile Asp Leu
               20                  25
<210> 143
   <211> 26
   <212> PRT
   <213> Artificial
<220>
   <223> künstliche Sekretionssequenz
<400> 143
   Met Lys Arg Ile Arg Ile Pro Met Thr Leu Ala Leu Ser Ala Ala Leu
   1               5                   10                  15
   Thr Ile Ala Pro Leu Ser Phe Ala Ser Ala
               20                  25
<210> 144
   <211> 26
   <212> PRT
   <213> Artificial
<220>
   <223> künstliche Sekretionssequenz
<400> 144
   Met Lys Lys Lys Gln Gln Ser Ser Ala Lys Phe Ala Val Ile Leu Thr
   1               5                   10                  15
   Val Val Trp Val Leu Leu Ala Ala Ile Val
               20                  25
<210> 145
   <211> 20
   <212> PRT
   <213> Artificial
<220>
   <223> künstliche Sekretionssequenz
<400> 145
   Met Arg Lys Tyr Thr Val Ile Ala Ser Ile Leu Ser Phe Leu Ser Val
   1               5                   10                  15
   Leu Ser Gly Gly
               20
<210> 146
   <211> 27
   <212> PRT
   <213> Artificial
<220>
   <223> künstliche Sekretionssequenz
<400> 146
   Met Lys Lys Ile Val Ser Ile Leu Phe Met Phe Gly Leu Val Met Gly
   1               5                   10                  15
   Phe Ser Gln Phe Gln Pro Ser Thr Val Phe Ala
               20                  25
<210> 147
   <211> 24
   <212> PRT
   <213> Artificial
<220>
   <223> künstliche Sekretionssequenz
<400> 147
   Met Lys Thr Leu Arg Thr Leu Cys Val Met Ile Leu Ser Gly Val Ile
   1               5                   10                  15
   Phe Phe Gly Leu Lys Ile Asp Ala
               20
<210> 148
   <211> 31
   <212> PRT
   <213> Artificial
<220>
   <223> künstliche Sekretionssequenz
<400> 148
   Met Lys Lys Leu Leu Thr Val Met Thr Met Ala Val Leu Thr Ala Gly
   1               5                   10                  15
   Thr Leu Leu Leu Pro Ala Gln Ser Val Thr Pro Ala Ala His Ala
               20                  25                  30
<210> 149
   <211> 28
   <212> PRT
   <213> Artificial
<220>
   <223> künstliche Sekretionssequenz
<400> 149
   Met Lys Arg Leu Leu Val Ser Leu Arg Val Trp Met Val Phe Leu Met
   1               5                   10                  15
   Asn Trp Val Thr Pro Asp Arg Lys Thr Ala Arg Ala
               20                  25
<210> 150
   <211> 22
   <212> PRT
   <213> Artificial
<220>
   <223> künstliche Sekretionssequenz
<400> 150
   Met Lys Val Phe Ile Ile Leu Gly Ala Ile Asn Ala Leu Leu Ala Val
   1               5                   10                  15
   Gly Leu Gly Ala Phe Gly
               20
<210> 151
   <211> 25
   <212> PRT
   <213> Artificial
<220>
   <223> künstliche Sekretionssequenz
<400> 151
   Met Lys Gly Asn Ile Tyr Ser Leu Phe Val Leu Ile Ala Ala Phe Phe
   1               5                   10                  15
   Trp Gly Thr Thr Gly Thr Val Gln Ala
               20                  25
<210> 152
   <211> 24
   <212> PRT
   <213> Artificial
<220>
   <223> künstliche Sekretionssequenz
<400> 152
   Met Lys Met Lys Ser Gly Met Glu Gln Ala Val Ser Val Leu Leu Leu
   1               5                   10                  15
   Leu Ser Arg Leu Pro Val Gln Ala
               20
<210> 153
   <211> 27
   <212> PRT
   <213> Artificial
<220>
   <223> künstliche Sekretionssequenz
<400> 153
   Met Lys Val Phe Ile Val Ile Met Ile Ile Val Val Ile Phe Phe Ala
   1               5                   10                  15
   Leu Ile Leu Leu Asp Ile Phe Met Gly Arg Ala
               20                  25
<210> 154
   <211> 28
   <212> PRT
   <213> Artificial
<220>
   <223> künstliche Sekretionssequenz
<400> 154
   Met Lys Lys Lys Gln Val Ser His Ala Ile Ile Ile Ser Val Met Leu
   1               5                   10                  15
   Ser Phe Val Ile Ala Val Phe His Thr Ile His Ala
               20                  25
<210> 155
   <211> 23
   <212> PRT
   <213> Artificial
<220>
   <223> künstliche Sekretionssequenz
<400> 155
   Met Lys Lys Arg Phe Ser Leu Ile Met Met Thr Gly Leu Leu Phe Gly
   1               5                   10                  15
   Leu Thr Ser Pro Ala Phe Ala
               20
<210> 156
   <211> 23
   <212> PRT
   <213> Artificial
<220>
   <223> künstliche Sekretionssequenz
<400> 156
   Met Lys Lys Leu Leu Ala Ala Gly Ile Ile Gly Leu Leu Thr Val Ser
   1 5 10 15
   Ile Ala Ser Pro Ser Phe Ala
   20
<210> 157
   <211> 27
   <212> PRT
   <213> Artificial
<220>
   <223> künstliche Sekretionssequenz
<400> 157
   Met Arg Lys Trp Tyr Phe Ile Leu Leu Ala Gly Val Leu Thr Ser Val
   1 5 10 15
   Ile Leu Ala Phe Val Tyr Asp Lys Thr Lys Ala
   20 25
<210> 158
   <211> 30
   <212> PRT
   <213> Artificial
<220>
   <223> künstliche Sekretionssequenz
<400> 158
   Met Gly Glu Ser Thr Ser Leu Lys Glu Ile Leu Ser Thr Leu Thr Lys
   1 5 10 15
   Arg Ile Leu Leu Ile Met Ile Val Thr Ala Ala Ala Thr Ala
   20 25 30
<210> 159
   <211> 28
   <212> PRT
   <213> Artificial
<220>
   <223> künstliche Sekretionssequenz
<400> 159
   Met Lys Phe Leu Leu Ser Val Ile Ala Gly Leu Leu Ile Leu Ala Leu
   1               5                   10                  15
   Tyr Leu Phe Trp Lys Val Gln Pro Pro Val Trp Ile
               20                  25
<210> 160
   <211> 30
   <212> PRT
   <213> Artificial
<220>
   <223> künstliche Sekretionssequenz
<400> 160
   Met Asn Lys Pro Thr Lys Leu Phe Ser Thr Leu Ala Leu Ala Ala Gly
   1               5                   10                  15
   Met Thr Ala Ala Ala Ala Gly Gly Ala Gly Thr Ile His Ala
               20                  25                  30
<210> 161
   <211> 22
   <212> PRT
   <213> Artificial
<220>
   <223> künstliche Sekretionssequenz
<400> 161
   Met Lys Phe Val Lys Ala Ile Trp Pro Phe Val Ala Val Ala Ile Val
   1               5                   10                  15
   Phe Met Phe Met Ser Ala
               20
<210> 162
   <211> 34
   <212> PRT
   <213> Artificial
<220>
   <223> künstliche Sekretionssequenz
<400> 162
   Met Asn Thr Leu Ala Asn Trp Lys Lys Phe Leu Leu Val Ala Val Ile
   1               5                   10                  15
   Ile Cys Phe Leu Val Pro Ile Met Thr Lys Ala Glu Ile Ala Glu Ala
               20                  25                  30
   Asp Thr
<210> 163
   <211> 39
   <212> PRT
   <213> Artificial
<220>
   <223> künstliche Sekretionssequenz
<400> 163
   Met Glu Glu Arg Ser Gln Arg Arg Lys Lys Lys Arg Lys Leu Lys Lys
   1               5                   10                  15
   Trp Val Lys Val Val Ala Gly Leu Met Ala Phe Leu Val Ile Ala Ala
               20                  25                  30
   Gly Ser Val Gly Ala Tyr Ala
           35
<210> 164
   <211> 41
   <212> PRT
   <213> Artificial
<220>
   <223> künstliche Sekretionssequenz
<400> 164
   Met Val Lys Ser Phe Arg Met Lys Ala Leu Ile Ala Gly Ala Ala Val
   1               5                   10                  15
   Ala Ala Ala Val Ser Ala Gly Ala Val Ser Asp Val Pro Ala Ala Lys
               20                  25                  30
   Val Leu Gln Pro Thr Ala Ala Tyr Ala
           35                  40
<210> 165
   <211> 26
   <212> PRT
   <213> Artificial
<220>
   <223> künstliche Sekretionssequenz
<400> 165
   Met Phe Asn Arg Leu Phe Arg Val Cys Phe Leu Ala Ala Leu Ile Met
   1               5                   10                  15
   Ala Phe Thr Leu Pro Asn Ser Val Tyr Ala
               20                  25
<210> 166
   <211> 32
   <212> PRT
   <213> Artificial
<220>
   <223> künstliche Sekretionssequenz
<400> 166
   Met Lys Trp Asn Asn Met Leu Lys Ala Ala Gly Ile Ala Val Leu Leu
   1               5                   10                  15
   Phe Ser Val Phe Ala Tyr Ala Ala Pro Ser Leu Lys Ala Val Gln Ala
               20                  25                  30
<210> 167
   <211> 26
   <212> PRT
   <213> Artificial
<220>
   <223> künstliche Sekretionssequenz
<400> 167
   Met Val Tyr Gln Thr Lys Arg Asp Val Pro Val Thr Leu Met Ile Val
   1               5                   10                  15
   Phe Leu Thr Leu Leu Ile Gln Ala Asp Ala
               20                  25
<210> 168
   <211> 30
   <212> PRT
   <213> Artificial
<220>
   <223> künstliche Sekretionssequenz
<400> 168
   Met Asn Lys Phe Leu Lys Ser Asn Phe Arg Phe Leu Leu Ala Ala Ala
   1               5                   10                  15
   Leu Gly Ile Ser Leu Leu Ala Ser Ser Asn Phe Ile Lys Ala
               20                  25                  30
<210> 169
   <211> 27
   <212> PRT
   <213> Artificial
<220>
   <223> künstliche Sekretionssequenz
<400> 169
   Met Lys Lys Trp Met Ile Thr Ile Ala Met Leu Ile Leu Ala Gly Ile
   1               5                   10                  15
   Ala Leu Phe Val Phe Ile Ser Pro Leu Lys Ser
               20                  25
<210> 170
   <211> 33
   <212> PRT
   <213> Bacillus amyloliquefaciens
<400> 170
   Met Phe Glu Lys Arg Phe Lys Thr Ser Leu Leu Pro Leu Phe Ala Gly
   1               5                   10                  15
   Phe Leu Leu Leu Phe His Leu Val Leu Ser Gly Pro Ala Ala Ala Asn
               20                  25                  30
   Ala
<210> 171
   <211> 947
   <212> DNA
   <213> Bacillus subtilis
<400> 171
   tgtcatcgta ccgtatttgt cgtaggcatt gtagagcttt cgcaaaatat catcttgttc 60
   cacaaattta tcgcctgttt ttctgatgaa aatctcattg tcactcagct tagtgacgag 120
   cgcctttcct gagctccacg catcgcccac tccagaaaaa cagctgctgt agcggggatc 180
   gcattgcaaa atcatctgcc ccagctccac atgcaggtcc ttatatttgt tttccatatc 240
   agttaattca gtgactacat catttgcaag ctgttcgagc ttatccggat caactgcaat 300
   gtttcctgac acaccgttca cctcccgagt ttatgtcttt cctctcttgc tttacgctcc 360
   agttcttcca ttctggcaat ttcagctttt cgttcttcta ttttgacagc ggcagccttc 420
   agctcttggc ttgtttcaag cagctcctgt tcgaattgat cgtaggcggc tttcgtttcc 480
   tcgaaatcct ggtcaaacga atctcttgct ttgccctgcc aagcatctcc caatgcagat 540
   aaccgggatt caagcagcat tttggagctt ctgacctctt ctgcggccgc actatacttg 600
   tttgccagct caatgacttt atatgagtcc aacttttgtc ccctgccttt tctaaattca 660
   cgcacaattg gatgttttat ataaatgatt ataaataatt cggcatgtat ccgaatcgta 720
   caaaagaacc ttttcataag aattggaagg gcgtatattc acttaaaatt cacagttggt 780
   gagactttaa gattacaaaa aaggtaaaaa aaccaaatct ctcagacata aggcaaatga 840
   gaaatttccc gctctatggg aaaaaacact aaagttgatc aaatgaccta agtgcgccaa 900
   acgtgttacg ggacgagcta tctcatggta taaatggaat tgtaaac 947
<210> 172
   <211> 997
   <212> DNA
   <213> Bacillus amyloliquefaciens
<400> 172
   acgggtgctg caggaacgga aagtcacccc ggtcggcggg acaaaagaaa ttcccatcga 60
   catcagaatc atcaccgccg cacacactga tctgagacag cttgtcgaaa gcaaaaagct 120
   gagagaagat ttattttacc ggcttcacgt ctgtccgatt catcttccgc cattaagaga 180
   acgcactgaa gacattcccg gcctcttctt tgaatttcag cgaaacagca ggtggccggg 240
   agagcttcct gatgactttt tagacatatt aaagggctgg aaatggccgg ggaatatcag 300
   agaattgttt aatgtgtttg aacgtctttc cgtcatgttc ccggacggac ggctgtccgg 360
   acgtcccctc gcatccctgc ttgaagcgtc aggtcttaaa gaggcggccc agccttttga 420
   acaagaagaa gcgcctcctg tctctatacg ggagcacatt cagaaaaaca tgatcgtaaa 480
   cgcgcttgaa tcggcgaagg gaaatgtcac gcaggccgcc aaaattgcgg ggattccgcg 540
   tagcacgttt tacaaacgcc tcaaaaaatt caaactcacc gccggacaat tgtaagaaac 600
   aagcatactt ctcttggctg ccggacacgt tatgtgtgag gcccaaagga ggtttgagac 660
   gtgaatacac agcgtgcgaa agacattgct gaatccgccg ccatggcgta tgtgacgtat 720
   gaaggagtcc cgatttatat tcagcatgtc aacgaagaca aagaaacagc caggattttt 780
   ccggtcggaa atcctcaatt tgagcaggaa gtgctgcttt ctgatttaca agagcatttt 840
   tgagaaaagg tctgctgatc aaagcagacc ttttatgttc ctgtgagaac tttcccgctt 900
   tttgagaaaa ataactaaaa atgattaaat gatctaactc ccccaaaccc ggtacagaga 960
   agccgttctc atgatataaa taaaatgtaa acgttat 997
<210> 173
   <211> 20
   <212> DNA
   <213> Artificial
<220>
   <223> Primer
<400> 173
   agtcacgacg ttgtaaaacg 20
<210> 174
   <211> 20
   <212> DNA
   <213> Artificial
<220>
   <223> Primer
<400> 174
   tgttgtgtgg aattgtgagc 20
<210> 175
   <211> 881
   <212> DNA
   <213> Bacillus subtilis
<400> 175
   gcatgcaaaa aatcaaataa ggagtgtcaa gaatgaggtt taataataaa atgttggcct      60
   tggcagcact actttttgca gcgcaagcgt cagcagacac gctcgaatca atagacaatt     120
   gcgcggtcgg atgtccaaca ggaggcagta gcaatgtctc tattgtcaga catgcttata     180
   cgttgaataa taacagtaca acaaagtttg ccaattgggt tgcatatcat attacaaaag     240
   acacgccagc aagcggaaag acaagaaatt ggaaaacaga tcctgctctc aatcctgcgg     300
   acactcttgc gcctgctgat tacacaggtg ccaatgccgc gcttaaagtc gatcgaggtc     360
   atcaagcgcc tcttgcatct cttgcgggag tttccgactg ggaatcgttg aactaccttt     420
   caaacatcac gccacaaaag tcagatttaa accagggagc ttgggctcgg ctggaagatc     480
   aggaacgtaa attaattgat cgtgctgata tctcatcggt gtatactgtt accgggccgc     540
   tgtatgagag agatatgggc aaattacctg gcactcagaa agcgcacacc atacccagcg     600
   cctactggaa ggtaattttt attaacaaca gcccggcggt taaccactat gcagcatttc     660
   ttttcgacca gaacacgccg aagggcgccg atttctgtca atttcgcgtg acggtggacg     720
   agatcgagaa aaggacagga ctgattatct gggccggtct gccggacgac gtgcaggctt     780
   ctctgaagag caaaccgggc gttctgccgg agttgatggg ctgcaaaaac tgacgaaaaa     840
   acccgccgaa gcgggtttat tcttcacgcg ggcgcctgca g                         881
<210> 176
   <211> 1066
   <212> DNA
   <213> Artificial
<220>
   <223> künstliche Nukleasesequenz inklusive Signalpeptid und Ribosomenbindungsstelle
<400> 176
   agtcacgacg ttgtaaaacg acggccagtg aattcgagct cggtacctcg cgaatgcatc     60
   tagatccaat gcatgcaaaa aatcaaataa ggagtgtcaa gaatgaggtt taataataaa    120
   atgttggcct tggcagcact actttttgca gcgcaagcgt cagcagacac gctcgaatca    180
   atagacaatt gcgcggtcgg atgtccaaca ggaggcagta gcaatgtctc tattgtcaga    240
   catgcttata cgttgaataa taacagtaca acaaagtttg ccaattgggt tgcatatcat    300
   attacaaaag acacgccagc aagcggaaag acaagaaatt ggaaaacaga tcctgctctc    360
   aatcctgcgg acactcttgc gcctgctgat tacacaggtg ccaatgccgc gcttaaagtc    420
   gatcgaggtc atcaagcgcc tcttgcatct cttgcgggag tttccgactg ggaatcgttg    480
   aactaccttt caaacatcac gccacaaaag tcagatttaa accagggagc ttgggctcgg    540
   ctggaagatc aggaacgtaa attaattgat cgtgctgata tctcatcggt gtatactgtt    600
   accgggccgc tgtatgagag agatatgggc aaattacctg gcactcagaa agcgcacacc    660
   atacccagcg cctactggaa ggtaattttt attaacaaca gcccggcggt taaccactat    720
   gcagcatttc ttttcgacca gaacacgccg aagggcgccg atttctgtca atttcgcgtg    780
   acggtggacg agatcgagaa aaggacagga ctgattatct gggccggtct gccggacgac    840
   gtgcaggctt ctctgaagag caaaccgggc gttctgccgg agttgatggg ctgcaaaaac    900
   tgacgaaaaa acccgccgaa gcgggtttat tcttcacgcg ggcgcctgca gattggatcg    960
   gatcccgggc ccgtcgactg cagaggcctg catgcaagct tggcgtaatc atggtcatag   1020
   ctgtttcctg tgtgaaattg ttatccgctc acaattccac acaaca                  1066
<210> 177
   <211> 147
   <212> DNA
   <213> Artificial
<220>
   <223> Multiple Klonierungsstelle (Multicloning-site)
<400> 177
   ggatcgatcc gcatgcgagc tcggtacccc gggtcgacct gcagccaagc ttaattagct     60
   gagcttggac tcctgttgat agatccagta atgacctcag aactccatct ggatttgttc    120
   agaacgctcg gttgccgccg ggcgttt                                        147
<210> 178
   <211> 28
   <212> DNA
   <213> Artificial
<220>
   <223> Primer
<400> 178
   cgccaaacgt gttacgggac gagctatc 28
<210> 179
   <211> 26
   <212> DNA
   <213> Artificial
<220>
   <223> Primer
<400> 179
   aaacgcccgg cggcaaccga gcgttc 26
<210> 180
   <211> 1047
   <212> DNA
   <213> Artificial
<220>
   <223> künstliche Nuklesasesequenz
<400> 180
   cgccaaacgt gttacgggac gagctatctc atggtataaa tggaattgta aacggatcga 60
   tccgcatgca aaaaatcaaa taaggagtgt caagaatgag gtttaataat aaaatgttgg 120
   ccttggcagc actacttttt gcagcgcaag cgtcagcaga cacgctcgaa tcaatagaca 180
   attgcgcggt cggatgtcca acaggaggca gtagcaatgt ctctattgtc agacatgctt 240
   atacgttgaa taataacagt acaacaaagt ttgccaattg ggttgcatat catattacaa 300
   aagacacgcc agcaagcgga aagacaagaa attggaaaac agatcctgct ctcaatcctg 360
   cggacactct tgcgcctgct gattacacag gtgccaatgc cgcgcttaaa gtcgatcgag 420
   gtcatcaagc gcctcttgca tctcttgcgg gagtttccga ctgggaatcg ttgaactacc 480
   tttcaaacat cacgccacaa aagtcagatt taaaccaggg agcttgggct cggctggaag 540
   atcaggaacg taaattaatt gatcgtgctg atatctcatc ggtgtatact gttaccgggc 600
   cgctgtatga gagagatatg ggcaaattac ctggcactca gaaagcgcac accataccca 660
   gcgcctactg gaaggtaatt tttattaaca acagcccggc ggttaaccac tatgcagcat 720
   ttcttttcga ccagaacacg ccgaagggcg ccgatttctg tcaatttcgc gtgacggtgg 780
   acgagatcga gaaaaggaca ggactgatta tctgggccgg tctgccggac gacgtgcagg 840
   cttctctgaa gagcaaaccg ggcgttctgc cggagttgat gggctgcaaa aactgacgaa 900
   aaaacccgcc gaagcgggtt tattcttcac gcgggcgcct gcagccaagc ttaattagct 960
   gagcttggac tcctgttgat agatccagta atgacctcag aactccatct ggatttgttc 1020
   agaacgctcg gttgccgccg ggcgttt 1047
<210> 181
   <211> 137
   <212> DNA
   <213> Artificial
<220>
   <223> Multiple Klonierungsstelle (Multicloning-site)
<400> 181
   gcatgcgagc tcggtacccc gggtcgacct gcagccaagc ttaattagct gagcttggac 60
   tcctgttgat agatccagta atgacctcag aactccatct ggatttgttc agaacgctcg 120
   gttgccgccg ggcgttt 137
<210> 182
   <211> 50
   <212> DNA
   <213> Artificial
<220>
   <223> Primer
<400> 182
   ggttctggca ggaccggcgg ctgcgtcagc agacacgctc gaatcaatag 50
<210> 183
   <211> 25
   <212> DNA
   <213> Artificial
<220>
   <223> Primer
<400> 183.
   cgcagccgcc ggtcctgcca gaacc 25
<210> 184
   <211> 176
   <212> DNA
   <213> Artificial
<220>
   <223> künstliche Sekretionssequenz
<400> 184
   cgccaaacgt gttacgggac gagctatctc atggtataaa tggaattgta aacggatccg     60
   catgcaaata aggagtgtca agaatgtttg caaaacgatt caaaacctct ttactgccgt    120
   tattcgctgg atttttattg ctgtttcatt tggttctggc aggaccggcg gctgcg        176
<210> 185
   <211> 85
   <212> DNA
   <213> Artificial
<220>
   <223> Primer
<400> 185
   ggttctggca ggaccggcgg ctgcgtcagc agaccaccat caccatcacc atggcggcga     60
   cacgctcgaa tcaatagaca attgc                                           85